# EUROPEAN PATENT SPECIFICATION

(11) **EP 1 518 936 B1**
(45) Date of publication and mention of the grant of the patent: **22.08.2007**
(21) Application number: 04022858.7
(22) Date of filing: 24.09.2004
(51) Int. Cl.: C12Q 1/68, C12N 9/02

(54) **Marker assisted selection of chicken against fishy taint**
Marker-unterstützte Auswahl von Hühnern gegen das "fish odour"- oder TMA-Syndrom
Sélection assistée par marqueurs de poulets contre la trimethylaminurie ou "fish odour syndrom"

(30) Priority: 24.09.2003 EP 03021556
(43) Date of publication of application: 30.03.2005
(73) Proprietor: Lohmann Tierzucht GmbH, 27454 Cuxhaven (DE)
(72) Inventor: Preisinger, Rudolf, 21789 Wingst (DE); Weigend, Steffen, 31535 Neustadt-Mariensee (DE); Vilkki, Johanna, 31630 Minkiö (FI)
(74) Representative: Vossius & Partner

(56) References cited:
- WO-A-03/057886
- DATABASE EMBL [Online] 20 February 2002 (2002-02-20), XP002268095 retrieved from EBI Database accession no. aj431390
- TREACY E P ET AL: "MUTATIONS OF THE FLAVIN-CONTAINING MONOOXYGENASE GENE (FMO3) CAUSE TRIMETHYLAMINURIA, A DEFECT IN DETOXICATION" HUMAN MOLECULAR GENETICS, OXFORD UNIVERSITY PRESS, SURREY, GB, vol. 7, no. 5, May 1998 (1998-05), pages 839-845, XP001053063 ISSN: 0964-6906
- ZSCHOCKE J ET AL: "Mild trimethylaminuria caused by common variants in FMO3 gene" LANCET, XX, XX, vol. 354, no. 9181, 4 September 1999 (1999-09-04), pages 834-835, XP004357828 ISSN: 0140-6736

## Description

The present invention relates to a method of testing an avian for its predisposition for fishy taint in eggs, comprising analyzing the nucleic acid of a sample comprising the gene encoding flavin-containing monooxygenase 3 (FM03) or a corresponding mRNA for nucleotide polymorphisms which are linked to said predisposition, wherein a difference from the wild-type FM03 sequence is indicative of fishy taint. The present invention also relates to a method of testing an avian for its predisposition for fishy taint in eggs comprising testing a sample for the presence or absence of a threonine in position 329 of FM03, wherein an absence of a threonine in said position is indicative for a predisposition to produce fishy tainted eggs or to pass on to the offspring a gene responsible for developing such a phenotype. Finally, the present invention relates to a kit for the detection of animals having a predisposition for fishy taint in eggs, comprising a nucleic acid molecule of wild-type FM03 or a fragment thereof, or a nucleic acid molecule differing from wild-type FM03 by at least one polymorphism of the present invention or a fragment of said nucleic acid molecule, or antibodies specific to FM03, in one or more container.

The economy of egg productions depends on the sensoric quality of the produced eggs. A fishy or crabby taint has frequently been detected in brown shelled eggs and may lead to considerable economic losses since even the occurrence of a low frequency of smelling eggs will lead to complaints by consumers.

The fishy taint occurs due to a substance, trimethylamine (TMA), in the egg yolk (Hobson *et al.,* 1973). TMA is a chemical substance [(CH₃)₃N] and is characterised by a very intensive smell of rotten fish. TMA enters the animal body through several different precursors contained in the diet, e.g., fish-meal, rapeseed or choline. Especially fish-meal contains large amounts of TMA-O, which can be easily converted to TMA (Pearson et al., 1983b). Rapeseed contains large amounts of Sinapine, another precursor of TMA, which is converted by Enterobacteria into TMA. In the physiological pathway TMA is metabolised by a liver enzyme, a TMA oxidase, which is responsible for the oxidation of TMA to TMA-O (Butler and Fenwick, 1984). TMA-O does not smell and leaves the body through the excrete. If the enzyme activity is reduced due to environmental or genetic influences not all or none of the TMA is converted to its odourless oxide. TMA remains in the body and is transferred into egg yolk. It is noteworthy that hens with this genetic defect also have a fishy breath. Remarkable environmental influences on the activity of TMA-oxidase exist as rapeseed is a rich source of tannin and goitrin which are able to inhibit the TMA-Oxidase (Pearson et al., 1981).

The genetic influence on TMO-Oxidase is displayed by the fact that only certain breeds are concerned for this problem (Hobson Frohock et al., 1973). Especially brown layers are affected (Pearson and Butler, 1979); for example Rhode Island Red and Light Sussex. No problems with tainting eggs have been reported for White Leghorn and New Hampshire. It has been suggested that the tainting problem is linked to shell color, however, Butler *et al.* (1984) could demonstrate that this is not the fact.

Within one breed or strain only certain hens lay tainting eggs. It is assumed that the fishy taint is an autosomally inherited trait which is caused by mutations on a single locus leading to a reduced capacity for TMA oxidation (Bolton et al., 1976, Pearson and Butler, 1979). Selection experiments have suggested that this trait is inherited with high heritability in both sexes (Pearson and Butler, 1983).

In commercial breeding programs scoring egg taint under challenge feeding conditions is used as input for selection to reduce this problem. Hens or other avians laying fishy eggs are identified by an organoleptical examination which is a rather subjective and time consuming effort.

The situation is quite different with respect to mammals, for which a number of markers associated with a similar fishy taint phenotype have been disclosed. In summary, most of the markers associated with fishy taint appear to reside in the gene encoding the flavin-containing monooxygenase 3 (FM03). This is not surprising since this protein has an enzymatic activity which is capable of oxidizing TMA to TMA-O.

In human, several mutations of FM03 have been identified as having an impact on the activity of the FM03 and, as a result, on the oxidation-process of TMA leading to different effects of the disease "Trimethylaminuria". Mutations leading to total loss of enzymatic activity, as well as moderate and silent mutations were reported by typing individuals suffering from fishy body odour.

The strongest impact on FM03 activity is caused by any mutation creating a stop codon so that the translation process of the protein is stopped too early and only a truncated protein is generated. In human this kind of mutation has been observed at the positions M66X G148X and E314X (http://human-fmo3.biochem.ucl.ac.uk/Human_FMO3/Tables/tableall.html). Remarkably, a similar mutation was identified in cattle, where the mutation at amino acid 238 creating a stop codon (R238X) is responsible for a fishy smell in milk (Lunden et al.2002 - Genome Res 12: 1885-88)

Rare cases in human have been detected in compound heterozygotes where two mutations, both heterozygous, were identified as having an effect on the enzyme activity (M66I and R492W; AKERMAN et al., 1999) if they are inherited as one haplotype. Moreover, in a few affected cases single mutations of FM03 have been observed in a heterozygous state. However the reason for their phenotypic effect remains to be elucidated (TREACY et al., 1998; DOLPHIN et al., 2000). The most common cases reported in human are mutations which are identified in the homozygous state leading to a permanent smell of the individuals (DOLPHIN et al., 1997; TREACY et al., 1998; AKERMAN et al., 1999). In humans, 12 missense mutations, three nonsense mutations and one deletion of FM03 are presently known to cause TMAuria (HERNANDEZ et al. (2003), CASHMAN et al. (2003)). In the human FM03 mutation database (http://human-fmo3.biochem.ucl.acuk/ Human_FM03) a majority of the known TMAuria causing mutations reside in exon7, but none of these within the -FATGY- motif.

Another mammalian species appears to have a similar association between FM03 mutation and fishy odour as can be deduced from the results recently disclosed in a PCT-application relating to fishy smell in cattle (WO 03/057886). In cattle, the causative mutation is a nonsense mutation in exon 6 of FM03 (R238X), causing premature termination of the protein before exon 7 (Lunden et al. 2002 - same publication) This is the only mutation of FM03 outside humans shown to lead to deficiency of TMA oxidation; it has been detected in one cattle breed, the Swedish Red and White. There is no published evidence in any other species on mutations of FM03 leading to fishy taint of excretions.

The outcome of the studies on human and cattle suggests that mutations (markers) associated with fishy smell in mammals generally reside in the FM03 gene and are likely to cause truncation of the encoded polypeptide. In view of the economic losses due to fishy smell of eggs, it would be desirable to be able to exclude animals with a predisposition to produce eggs with fishy smell from breeding programs.

As is well known in the art, taxonomic differences between mammals and avians are quite significant. In this regard, the FM03 gene does not represent an exception. This is reflected by a comparison of the amino acid sequence of the chicken FM03 gene and the bovine and human FMO3 gene, yielding levels of sequence identity of 61% between the chicken and human sequences, and 64% sequence identity between the chicken and bovine sequences. Therefore, it appears unlikely that the teaching based on mammals can simply be applied to the avian family. Moreover, it needs to be stressed, that the disease manifestation in avians is quite different in the sense that the tainting is observed only under certain conditions, i.e. when the enzymatic mechanism of the hen is overloaded. Feed can affect the trait by providing increased dietary TMA levels or precursors for TMA formation by intestinal microbes or inhibitors of the oxidase. Known precursors in chicken for TMA are e.g. TMA-oxide, betaine, choline and sinapine. Polyphenolic tannins and goitrins have been described as inhibitors of TMA oxidation. Organoleptic detection of tainting eggs (or chicken breath) has been used in breeding programs to reduce the occurrence of the trait. However, as mentioned, the method requires feeding challenges and is problematic because it is dependent on the observer as well as environmental factors.

At present no reliable method exists for testing avian animals for their predisposition to produce fishy taint in eggs as no genetic marker correlating with fishy odour is available.

Thus, the technical problem underlying the present invention was to provide means and methods for testing an avian for its predisposition to produce eggs with fishy taint. The solution to this technical problem is achieved by providing the embodiments characterized in the claims. The invention is defined by the claims.

Accordingly, the present invention relates to a method of testing an avian for its predisposition for fishy taint in eggs comprising analyzing the nucleic acid of a sample comprising the gene encoding FM03 or a corresponding mRNA for a nucleotide polymorphism which is linked to said predisposition, wherein a serine codon in a position corresponding to position 329 of chichen FMO3 as schown in SEQ ID NO:25 or in a homologous position of other avian FMO3 (poly)peptides is indicative of fishy taint.

The term "predisposition" refers to a genetic precondition to produce eggs with fishy odour or to pass on to the offspring specific alleles or variants of a gene responsible for developing such a phenotype.

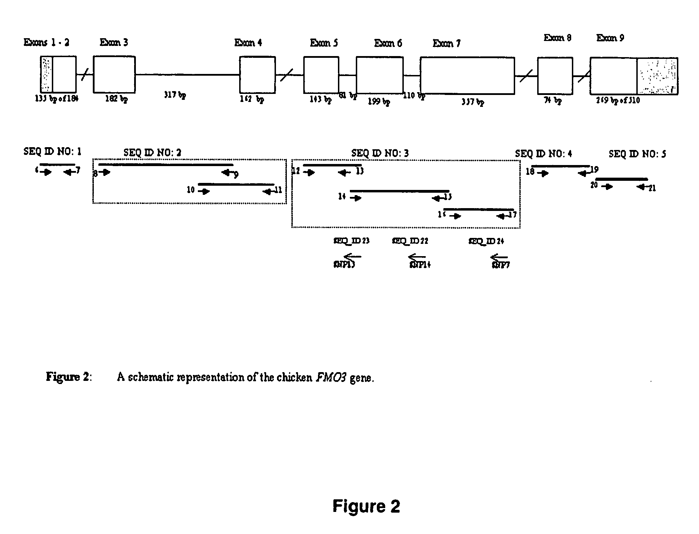

The terms "flavin-containing monooxygenase 3" or "FM03" relate to (poly)peptides with enzymatic activity which are capable of oxidizing TMA to TMA-O. In the case of chicken, FMO3 is encoded, for example, by the nucleic acid molecule deposited under Genebank accession number AJ431390.1. For the purpose of the present invention chicken wild-type FM03 is represented by SEQ ID NOs: 1 to 5, arranged as shown in Figure 2. The polymorphisms disclosed in the present invention are designated in SEQ ID NOs: 1 to 5 as "variations". Trait associated variants are summarized in table 2.

The term "(poly)peptide" refers alternatively to peptide or to polypeptides. Peptides conventionally are covalently linked amino acids of up to 30 residues, whereas (poly)peptides (also referred to as "proteins") comprise 31 and more amino acid residues.

The term "FMO3 with enzymatic activity" refers to a biologically active FM03 (poly)peptide. Biologically active relates to the enzymatic function of FM03 which can be assayed as disclosed in Störmer et al., 2000, J. of Clinical Pharmacology, 50: 553-561 or as described in Example 1 of the present invention.

As used herein, the term "avian" includes for example chicken, turkey, duck, goose, quail, pheasants, parrots, finches, hawks, crows and ratites including ostrich, emu and cassowary.

The term "fishy taint" or "fishy odour" refers to taint or odours created by trimethylamine (TMA). Fishy taint may be detected by simple smelling. However, alternatively the amount of TMA may be determined by analytical analysis, for example by the method describe in Example 1 of the present invention.

As used throughout the present invention the term "sample" relates to a specimen taken from egg, feather, blood, semen, skin, nail or tissues obtainable from liver or kidney.

The term "gene encoding FM03" refers to the genomic DNA sequence from which wild-type or variant FM03 is expressed. The term "corresponding mRNA" relates to mRNA transcribed from said gene or from a cDNA of FM03.

Said testing or assaying may be performed by PCR, Southern and Northern Blotting (Sambrook et al., "Molecular Cloning, A Laboratory Manual"; ISBN: 0879695765, CSH Press, Cold Spring Harbor, 2001) but may also comprise "ligase chain reaction"(LCR, EPA320308), "cyclic probe reaction" (CPR), "strand displacement amplification" (SDA, Walker et al. 1992, Nucleic Acid Res. (7): 1691-1696), "transcription based amplification systems" (TAS, Kwoh et al. 1989, Proc. Nat. Acad. Sci. USA 86: 1173, Gingeras et al., PCT Application WO 88/10315), allele-specific hybridization, allele-specific primer extension, allele-specific oligonucleotide ligation, allele-specific cleavage of a flap probe, Taqman PCR, Pyrosequencing, Snapshot, Restriction Fragment length Polymorphism Analysis (RFLP) or Maldi-Tof.

Particularly preferred according to the present invention is the testing comprising a step of amplification of the nucleic acid molecule. In the present invention, the term "amplification" or "amplify" means increase in copy number. The person skilled in the art knows various methods to amplify nucleic acid molecules. Amplification methods include, but are not limited to, "polymerase chain reaction" (PCR), "ligase chain reaction"(LCR, EPA320308), "cyclic probe reaction" (CPR), "strand displacement amplification" (SDA, Walker et al. 1992, Nucleic Acid Res. (7): 1691-1696), "transcription based amplification systems" (TAS, Kwoh et al. 1989, Proc. Nat. Acad. Sci. USA 86: 1173, Gingeras et al., PCT Application WO 88/10315).

Preferably, amplification of DNA is accomplished by using polymerase chain reaction (PCR). The PCR consists of many repetitions of a cycle which consists of: (a) a denaturation step, which melts both strands of a DNA molecule; (b) an annealing steep, which is aimed at allowing the primers to anneal specifically to the melted strands of the DNA molecule; and (c) an extension step, which incorporates to the primers complementary to those of the strand of DNA to which the primers are annealed. The concentrations of primers, nucleotidetriphosphates, enzyme and buffers used will be apparent from and include the process parameters described in the Examples that follow. However, generally, PCR can be performed for example in a 50 µl reaction mixture containing 5 µl of 10 x PCR buffer with 1.5mM mM MgCl₂, 200 µM of each deoxynucleoside triphosphate, 0.5 µl of each primer (10 µM), 0.5 µl, 30 ng of template DNA and 2.5 Units of Taq Polymerase. The primers for the amplification may be labelled or be unlabelled. DNA amplification can be performed, e.g., with a model 2400 thermal cycler (Applied Biosystems, Foster City, CA): 2 min at 94°C, followed by 35 cycles consisting of annealing (30 s at 50°C), extension (1 min at 72°C), denaturation (10 s at 94°C) and a final annealing step at 55°C for 1 min as well as a final extension step at 72°C for 5 min. However, the person skilled in the art knows how to optimize these conditions for the amplification of specific nucleic acid molecules (see for example Sambrook et al., "Molecular Cloning, A Laboratory Manual"; ISBN: 0879695765, CSH Press, Cold Spring Harbor, 2001).

A further method of nucleic acid amplification is the "reverse transcriptase polymerase chain reaction" (RT-PCR). This method is used when the nucleic acid to be amplified consists of RNA. The term "reverse transcriptase" refers to an enzyme that catalyzes the polymerisation of deoxyribonucleoside triphosphates to form primer extension products that are complementary to a ribonucleic acid template. The enzyme initiates synthesis at the 3'-end of the primer and proceeds toward the 5'-end of the template until synthesis terminates. Examples of suitable polymerizing agents that convert the RNA target sequence into a complementary, copy-DNA (cDNA) sequence are avian myeloblastosis virus reverse transcriptase and Thermus thermophilus DNA polymerase, a thermostable DNA polymerase with reverse transcriptase activity marketed by Perkin Elmer. Typically, the genomic RNA/cDNA duplex template is heat denatured during the first denaturation step after the initial reverse transcription step leaving the DNA strand available as an amplification template. Suitable polymerases for use with a DNA template include, for example, E. coli DNA polymerase I or its Klenow fragment, T.sub.4 DNA polymerase, Tth polymerase, and Taq polymerase, a heat-stable DNA polymerase isolated from Thermus aquaticus and developed and manufactured by Hoffmann-La Roche and commercially available from Perkin Elmer. The latter enzyme is widely used in the amplification and sequencing of nucleic acids. The reaction conditions for using Taq polymerase are known in the art and are described, e.g., PCR Technology, Erlich, H. A. 1989. Stockton Press, New York or Innis, M. A., D. H. Gelfand, J. J. Sninsky, and T. J. White. 1990. PCR Protocols: A guide to methods and applications. Academic Press, New York.

High-temperature RT provides greater primer specificity and improved efficiency. Copending U.S. patent application Serial No. 07/746, 121, filed Aug. 15, 1991, describes a "homogeneous RT-PCR" in which the same primers and polymerase suffice for both the reverse transcription and the PCR amplification steps, and the reaction conditions are optimized so that both reactions occur without a change of reagents. Thermus thermophilus DNA polymerase, a thermostable DNA polymerase that can function as a reverse transcriptase, can be used for all primer extension steps, regardless of template. Both processes can be done without having to open the tube to change or add reagents; only the temperature profile is adjusted between the first cycle (RNA template) and the rest of the amplification cycles (DNA template). The RT Reaction can be performed, for example, in a 20ul reaction mix containing:4 ul of 5x ANV-RT buffer 4 ul, 2 ul of Oligo dT(100 ug/ml), 2ul of 10 mM dNTPs, 1 ul total RNA,10 Units of AMV reverse transcriptase and H2O to 20 ul final volume. The reaction may be, for example, performed by using the following conditions: The reaction is held at 70 C° for 15 minutes to allow for reverse transcription. The reaction temperature is then raised to 95 C° for 1 minute to denature the RNA-cDNA duplex. Next, the reaction temperature undergoes two cycles of 95°C for 15 seconds and 60 C° for 20 seconds followed by 38 cycles of 90 C° for 15 seconds and 60 C° for 20 seconds. Finally, the reaction temperature is held at 60 C° for 4 minutes for the final extension step, cooled to 15 C°, and held at that temperature until further processing of the amplified sample.

SEQ ID NOs: 1 to 5 disclosed in the present invention, relate to nucleic sequences of FM03 comprising, in addition to the coding or exon sequence of chicken FM03, further nucleotide sequences from the non-coding region of FM03, including sequence information derived from introns of FM03. Figure 2 shows a representation of the relative position of SEQ ID NOs: 1 to 5 with respect to the PCR primers of the present invention and the genomic organization of FM03.

**Table 1 shows the exon positions of chicken FMO3 (SEQ ID NOs.: 1 to 5) and the nucleotide numbering.**

| SEQ ID Nos | exon # | Nucleotides |
|---|---|---|
| 1 | 1 and 2 | 1-135 |
| 2 | 3 | 1-182 |
| 2 | 4 | 500-662 |
| 3 | 5 | 1-143 |
| 3 | 6 | 226-425 |
| 3 | 7 | 537-894 |
| 4 | 8 | 1-74 |
| 5 | 9 | 3-335 |

For the purpose of the present invention, the sequences of SEQ ID NOs 1 to 5 represent "wild-type FM03". Polymorphisms of this sequence are indicated as "variations" where the direction is from wild-type → mutant. The polypeptide of SEQ ID NO: 25 corresponds to the "mutant polypeptide" (with two unknown amino acids at positions 47 and 48) having a serine at position 329.

The terms "flavin contain monooxygenase 3" and "FM03" also relate to these variants and alleles of FM03, but also refer to splicing variants. The numbering as used throughout the present invention relates to SEQ NO: 1 to 5 for nucleic acid molecules and SEQ ID NO: 25 for (poly)peptides. In addition, the term "FMO3" relates to species homologues in other avian animals including chicken, turkey, duck, goose, quail, pheasants, parrots, finches, hawks, crows and ratites including ostrich, emu and cassowary. Said alleles or variants encode a (poly)peptide which may be a wild-type FM03 (poly)peptide, however the term "variant" also refers to (poly)peptides comprising (poly)peptide sequence differing from wild-type FM03. Also comprised by the term "variant" are recombinant variants of FM03, fragments of FM03 and fusion proteins comprising FM03 (poly)peptide sequences. Said variant may be an N-terminally or C-terminally truncated FM03 or an FM03 containing one or more N- or C-terminal additions of amino acid residues.

The term "nucleic acid" or "nucleic acid molecule" refers to DNA or RNA or modified DNA or RNA but also refers to nucleic acid analogs. A number of modifications have been described that alter the chemistry of the phosphodiester backbone, sugars or heterocyclic bases. Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include, but are not limited to, 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire phosphodiester backbone with a peptide linkage. Sugar modifications are also used to enhance stability and affinity. The a-anomer of deoxyribose may be used, where the base is inverted with respect to the natural b-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

Sequence identity may be determined by using the Bestfit® program (Wisconsin Sequence Analysis Package, Version 8 for Unix, Genetics Computer Group, University Research Park, 575 Science Drive, Madison, WI 53711). Bestfit® uses the local homology algorithm of Smith and Waterman to find the best segment of homology between two sequences *(*Advances in Applied Mathematics 2:482-489 (1981)). When using Bestfit® or any other sequence alignment program to determine whether a particular sequence is, for instance, 95% identical to a reference sequence, the parameters are set, of course, such that the percentage of identity is calculated over the full length of the reference nucleotide sequence and that gaps in homology of up to 5% of the total number of nucleotides in the reference sequence are allowed. The identity between a first sequence and a second sequence, also referred to as a global sequence alignment, is determined using the FASTDB computer program based on the algorithm of Brutlag and colleagues (Comp. App. Biosci. 6:237-245 (1990)). In a sequence alignment the query and subject sequences are both DNA sequences. An RNA sequence can be compared by converting U's to T's. The result of said global sequence alignment is in percent identity. Preferred parameters used in a FASTDB alignment of DNA sequences to calculate percent identity are: Matrix=Unitary, k-tuple=4, Mismatch Penalty=1, Joining Penalty=30, Randomization Group Length=0, Cutoff Score=1, Gap Penalty=5, Gap Size Penalty 0.05, Window Size=500 or the length of the subject nucleotide sequence, whichever is shorter.

Particularly based on the methods of the present invention, it was discovered for the first time that specific variants of the FM03 gene in chicken are associated with the TMA content of the egg yolk, which in turn is associated with the fishy odour of egg yolk in chicken fed with rapeseed meal. Amongst others, a polymorphism in the FM03 gene has been discovered affecting a conserved amino acid of the encoded FM03 (poly)peptide (see table 2).

**Table 2: Detected polymorphisms between tainting and non-tainting individuals**

| *In coding regions:* | | | | | |
|---|---|---|---|---|---|
| ' #SNP' | SEQ_ID and nt position | #exon | Wild-type chromosome | 'tainting' chromosome | Amino acid |
| #1 | 1 / nt 90 | 2 | C | T | C |
| #2 | 2/nt86 | 3 | C | T | P |
| #3 | 3/nt98 | 5 | T or C | C | N |
| #4 | 3/nt101 | 5 | T or C | C | S |
| #5 | 3/nt249 | 6 | C | T | G |
| #6 | 3/nt615 | 7 | C | T | N |
| **# 7** | **3/ nt 694** | **7** | **A** | **T** | **T → S** |
| #8 | 5/nt60 | 9 | T OR C | C | T |
| # 9 | 5 / nt 150 | 9 | T | C | P |

| *In non-coding regions:* | | | | |
|---|---|---|---|---|
| ' #SNP' | SEQ_ID and nt position | # intron | Wild-type chromosome | 'tainting chrom.' |
| #10 | 2 / nt 689 | 3 | G | A |
| #11 | 3/nt184 | 4 | A or G | G |
| # 12 | 3/nt 189 | 4 | A or G | G |
| #13 | 3/nt191 | 4 | G | C |
| #14 | 3 / nt 486 | 5 | G or A | A |
| #15 | 3 / nt 510 | 5 | G or C | C |
| #16 | 4/nt85 | 7 | T | C |
| #17 | 4 / nt 277 | 7 | C | T |

The discovery of the present invention allows to reliably predict which animals in a chicken farm or poultry breeding program have a predisposition to produce eggs with fishy odour and/or may pass on this predisposition to their offspring.

Marker assisted selection, which provides the ability to select against a specific unfavorable recessive allele, involves the identification of a molecular marker that co-segregates with the allele causing the defect. Nucleic acid markers have several advantages, being relatively easy to measure and unambiguous. When using codominant DNA markers heterozygous and homozygous animals can be distinctively distinguished, which enables identification of carriers of a recessive unfavorable allele. Once a marker system is established, selection decisions can be made easily because nucleic acid markers can be assayed at any time after the nucleic acid-containing sample has been collected from an individual, regardless of the expression of the trait (e.g. for egg quality related traits, from cocks and chicks not laying eggs).

In a preferred embodiment of the present invention's method of testing an avian for its predisposition for fishy taint in eggs, said wild-type FM03 sequence is represented in part by any one of SEQ ID NOs: 1 to 5. Accordingly, the FM03 amino acid sequence may be entirely of wild-type FM03, corresponding to the polypeptide encoded by the nucleic acid molecule deposited under databank accession number AJ431390.

The specification of the present invention describes that in the present invention's method of testing, said difference may also comprise at least one polymorphism selected from the list of single nucleotide polymorphisms as shown in table 2.

Also disclosed herein is that the polymorphisms may be genetically linked as a haplotype. Genetic screening (also called genotyping or molecular screening), can be broadly defined as testing to determine if an individual has mutations (alleles or polymorphisms) that either cause a specific phenotype or are "linked" to the mutation causing the phenotype. Linkage refers to the phenomenon that the DNA sequences which are close together in the genome have a tendency to be inherited together. Two or more sequences may be linked because of some selective advantage of co-inheritance. More typically, however, two or more polymorphic sequences are co-inherited because of the relative infrequency with which meiotic recombination events occur within the region between the two polymorphisms. The co-inherited polymorphic alleles are said to be in linkage disequilibrium with one another because, in a given population, they tend to either both occur together or else not occur at all in any particular member of the population. Indeed, where multiple polymorphisms in a given chromosomal region are found to be in linkage disequilibrium with one another, they define a quasi-stable genetic "haplotype." Furthermore, where a phenotype-causing mutation is found within or in linkage with this haplotype, one or more polymorphic alleles of the haplotype can be used as a diagnostic or prognostic indicator of the likelihood of developing a specific phenotype. Identification of a haplotype which spans or is linked to a phenotype-causing mutational change, serves as a predictive measure of an individual's likelihood of having inherited that phenotype-causing mutation. Importantly, such prognostic or diagnostic procedures can be utilized without necessitating the identification and isolation of the actual phenotype-causing molecule. This is significant because the precise determination of the molecular basis of the establishment of a specific phenotype can be difficult and laborious, especially in the case of multifactorial phenotype.

The presence of a codon other than a threonine codon in position 329 of chicken FM03 or in a homologous position of other avian FMO3 (poly)peptides is indicative of fishy taint in eggs. Said codon in position 329 of chicken FMO3 may encode an alanine, cysteine, aspartate, glutamate, phenylalanine, glycine, histidine, isoleucine, lysine, leucine, methionine, asparagine, proline, glutamine, arginine, serine, valine, tryptophan or tyrosine. Suitable methods and conditions for detecting very small differences between two closely related nucleic acid molecules have been reported in the art (see Itakura, K., J. Rossi, and R. B. Wallace. 1984. Synthesis and use of synthetic oligonucleotides. Annual Review of Biochemistry, 53:323-356). In fact, it has been shown that the sensitivity of these methods allows to detect a single nucleotide exchange.

In another preferred embodiment of the present invention, said testing comprises (a) hybridizing a probe comprising the polymorphic position under stringent conditions to the FMO3 gene or to a transcription product therefrom, wherein the conditions of hybridization are such that binding can only occur if the sequence of the probe and the target nucleic acid at the polymorphic position are completely complementary and (b) detecting whether hybridization has occurred, wherein hybridization of a probe comprising the polymorphic position is indicative for a predisposition for fishy taint in eggs.

The term "hybridizes under stringent conditions", as used throughout the description of the present invention, is well known to the skilled artesian and corresponds to conditions of high stringency. Appropriate stringent hybridization conditions for each sequence may be established by a person skilled in the art by modifying parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1x SSC at about 65°. Other stringent hybridization conditions are for example 0.2 x SSC (0.03 M NaCl, 0.003M Natriumcitrat, pH 7) at 65°C. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility. Also contemplated are nucleic acid molecules encoding (poly)peptides with FM03 activity, wherein the nucleic acid molecules hybridize to the nucleic acid molecule encoding the FM03 activity at lower stringency hybridization conditions. Changes in the stringency of hybridization and signal detection are, for example, accomplished through the manipulation of formamide concentration (lower percentages of formamide result in lowered stringency); salt conditions, or temperature. For example, lower stringency conditions include an overnight incubation at 37 degree °C in a solution comprising 6X SSPE (20X SSPE = 3M NaCl; 0.2M NaH₂PO₄; 0.02M EDTA, pH 7.4), 0.5% SDS, 30% formamide, 100 ug/ml salmon sperm blocking DNA; followed by washes at 50 degree °C with 1XSSPE, 0.1% SDS. In addition, to achieve even lower stringency, washes performed following stringent hybridization can be done at higher salt concentrations (e.g. 5X SSC). Note that variations in the above conditions may be accomplished through the inclusion and/or substitution of alternate blocking reagents used to suppress background in hybridization experiments. Typical blocking reagents include, but are not limited to, Denhardt's reagent, BLOTTO, heparin, denatured salmon sperm DNA, and commercially available proprietary formulations. The inclusion of specific blocking reagents may require modification of the hybridization conditions described above, due to problems with compatibility.

The choice and design of the probe particularly depends on the base composition of the target nucleic acid molecule to which the probe hybridizes. Generally, a probe is designed so that the presence of as few as one mismatch in the base pairing of the probe with the target nucleic acid molecule is detectable. Typically, the nucleotide sequence of such a probe corresponds to a nucleotide sequence in a region of FM03 suspected of containing a mutation. Preferentially, the probe comprises at least one of the polymorphic positions of table 2. However, the test may also comprise hybridizing a probe of wild-type sequence to an exon or intron sequence of FM03. In this case, an absence of hybridization is indicative for a predisposition for fishy taint in eggs, whereas hybridization would identify an animal with no predisposition for eggs with fishy odour.

The person skilled in the art knows various techniques for detecting mismatches in the base pairing of nucleic acid molecules. Preferably, these techniques include, but are not limited to, electrophoretic mobility shift assays or thermal mobility shift assays. Another preferred method is a mutation-sensitive PCR. The term "mutation-sensitive PCR" relates to a PCR approach comprising the use of primers which either allow or prevent the generation of amplification products. According to this technique, primers comprising at least one of the polymorphic positions of table 2 would not be capable of binding to wild-type FM03. Hence, the absence of an amplification product would be indicative for eggs with no fishy odour. On the other hand, if the primers are of wild-type sequence, the presence of an amplification product would be indicative for eggs with no fishy odour.

In another more preferred embodiment of the present invention, said probe is detectably labeled. Any of the preferred labels of the present invention (see above) may be used for labeling the probe. However, a reporter dye and a quenching dye are particularly preferred according to the present invention.

In another preferred embodiment of the present invention said testing comprises determining the nucleic acid sequence of at least a portion of said nucleic acid molecule. The nucleic acid sequence may be determined by using any of the nucleic acid sequencing techniques known in the art (see Sambrook et al., "Molecular Cloning, A Laboratory Manual"; ISBN: 0879695765, CSH Press, Cold Spring Harbor, 2001). However, particularly preferred according to the present invention are the methods of Sanger and Maxam/Gilbert.

In another preferred embodiment of the present invention, the determination of the nucleic acid sequence is effected by solid-phase minisequencing. The solid-phase minisequencing method is designed to detect single base changes or small deletions and insertions in amplified DNA. Solid-phase refers to the solid matrix employed to immobilise the target DNA allowing a minisequencing reaction to take place. The detection of the alteration in the base sequence occurs on the immobilised sequence by incorporation of a single residue of a labelled nucleotide. Typically streptavidin coated magnetic beads or streptavidin coated wells in microtitreplates provide a suitable support, taking advantage of the interaction between biotin and avidin. For diagnostic purposes microtitreplates are preferred as they facilitate large-scale population screening. The magnetic bead approach may be used in combination with microtitreplates.

Typically, a DNA fragment under investigation for a known mutation is enzymatically amplified by using one unbiotinylated and one 5'biotinylated primer. The PCR product carrying the biotin labelled residue is then captured at the 5' end onto the steptavidin coated solid matrix. The non-biotinylated strand and excess primers are eluted under alkaline conditions leaving the single stranded template accessible for the primer extension reaction. A specific sequencing primer is to anneal to the target DNA immediately upstream of the base change in the sequence. Primer elongation of the target DNA sequence occurs in the presence of Taq polymerase and a mixture of dNTPs and ddNTPS. Usually two different labelled dNTPs are required to detect the nucleotide variation. This allows identification of nucleotide substitutions in samples from both heterozygous and homozygous individuals. A homozygous individual will generate only one signal corresponding to the nucleotide present whereas a heterozygote will produce a signal from the reaction with both labelled dNTPs. The dNTPs may be labelled radioactively using [³H] or [³²P] or non-radioactively with digoxygenin or fluorescein. Inclusion of the ddNTPs is necessary for complete termination of the reaction. For instance to detect variants in FM03, [³H]dTTP and ddNTP are added for the T-reaction. The normal sequence is revealed by adding [³H]dATP with ddNTP for the A-reaction. The radioactive emissions are counted in scintillation fluid in a liquid scintillation counter. If nonradioactive labelling is utilised the sequencing reaction requires a long incubation stage followed by several washing steps. Incorporation of the labelled nucleotide is detected through immunological reaction with an enzyme labelled anti-hapten conjugate and a substrate. Genotyping can be achieved by comparing photochemical signals or radioactive emissions. For instance with the fishy egg taint associated polymorphism, incorporation of [³H]dATP only occurs in the wild-type allele and [³H]dTTP incorporation identifies the variant allele. The solid-phase minisequencing method described here can be applied to any known point mutation, deletion or insertion. The same reaction conditions can be used to detect several mutations. The main drawback is the inability to perform multiple analyses in a single reaction. However safer and more cost-effective methods, which are preferred methods according to the present invention, have been developed including RFLP analysis and ARMS PCR and more recently automated fluorescent sequencing. These methods are more efficient at picking up known mutations and therefore more suitable for diagnostic screening purposes.

In another preferred embodiment of the present invention the method of testing further comprises, prior to analyzing the nucleic acid, amplification of at least a portion of said nucleic acid. Amplification may be performed by any of the amplification methods described in the present invention, however, PCR-based amplification are preferred in accordance with the present invention.

In another preferred embodiment of the present invention, primers consisting of SEQ ID NOs: 6 to 24 are used for amplification.

In a more preferred embodiment of the present invention said amplification is effected by or said amplification is the polymerase chain reaction (PCR).

In a preferred embodiment of the present invention, the nucleic acid is analyzed by the use of: (a) a primer extension assay; (b) a Taqman^{®} PCR, (c) a differential hybridization assay; (d) an assay which detects allele-specific enzyme cleavage; and/or allele specific PCR. Alternatively, the nucleic acid molecule can be analyzed by any of the methods described further below.

In another preferred embodiment of the present invention's method of testing an avian for its predisposition for fishy taint in eggs, prior to analysing the nucleic acid, at least a portion of said nucleic acid is amplified by PCR, the method comprising cycles of (a) stringent hybridization with at least two primers sufficiently complementary to bind to the target FM03 DNA under stringent conditions of hybridization, wherein at least one primer comprises at least one polymorphic position indicated in table 2, having in the polymorphic position of the FM03 gene the sequence of a variant and not of wild-type FM03; and (b) strand elongation and denaturing, wherein (i) a failure to generate a PCR product is indicative for a predisposition for eggs with no fishy taint and (ii) the generation of a PCR product is indicative for a predisposition for eggs with fishy taint.

In another preferred embodiment of the present invention's method of testing an avian for its predisposition for fishy taint in eggs, prior to analysing the nucleic acid, at least a portion of said nucleic acid is amplified by PCR, the method comprising cycles of (a) stringent hybridization with at least two primers sufficiently complementary to bind to the target FM03 DNA under stringent conditions of hybridization, wherein at least one primer comprises at least one polymorphic position indicated in table 2, but having the sequence of wild-type FM03 in said position; and (b) strand elongation and denaturing, wherein (i) a failure to generate a PCR product is indicative for a predisposition for fishy taint in eggs and (ii) the generation of a PCR product is indicative for a predisposition for eggs with no fishy taint.

The present invention also relates to a primer suitable for use in detecting the polymorphism at nucleotide position 694 of SEQ ID NO:3 as shown in table 2 said primer consisting of a nucleotide sequence of about at least 8 to about 30 nucleotides capable of hybridizing, preferably under stringent conditions to the nucleotide sequence of SEQ ID NO: 3 and said primer comprising a T at position 694 of SEQ ID NO: 3. The specification describes that the primer may also be a nucleic acid molecule comprising the nucleotide sequence of any one of SEQ ID NOs: 6 to 24 or any other primer comprising 15 or more nucleotides of FMO3 and capable of specifically hybridizing to FM03 under stringent conditions.

The term "primer" or "oligonucleotide" refers to a short nucleic acid molecule from about 8 to about 30 nucleotides in length, whether natural or synthetic, capable of acting as a point of initiation of DNA synthesis under conditions in which synthesis of a primer extension product complementary to a nucleic acid strand is induced, i.e., in the presence of four different nucleoside triphosphates or analogues thereof and an agent for polymerisation (i.e., DNA polymerase or reverse transcriptase) in an appropriate buffer and at a suitable temperature. A primer is a nucleic acid molecule as described above. Preferably, a primer is a single-stranded oligodeoxyribonucleotide. The appropriate length of a primer depends on the intended use of the primer but typically ranges for PCR primers and primers used in sequencing reactions from 10 to 30 nucleotides. Short primer molecules generally require cooler temperatures to form sufficiently stable hybrid complexes with the template. A primer need not reflect the exact sequence of the template but must be sufficiently complementary to hybridize with a template. "Hybridize" refers to the binding of two single stranded nucleic acids via complementary base pairing, i.e. A to T (in RNA: U), G to C. The term "primer pair" refers to two primers that hybridize with the + and - strand, respectively, of a double stranded nucleic acid molecule, and allow the amplification of DNA fragments, as for example in a PCR reaction. A primer can be labeled, if desired, by incorporating a compound detectable by spectroscopic, photochemical, biochemical, immunochemical, or chemical means. Preferred labels of nucleic acid molecules and of (poly)peptides of the present invention include, but are not limited to, fluorescent dyes, electron-dense reagents, biotin, or small peptides for which antisera or monoclonal antibodies are available. A label can also be used to "capture" the primer, so as to facilitate a selection of amplified nucleic acid or fragments thereof. Carboxyfluorescein (FAM) and 6-carboxy-X-rhodamine (ROX), are preferred labels of nucleic acid molecules and (poly)peptides. However, other preferred labels include fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the DNA is conjugated to biotin, haptens, etc. having a high affinity binding partner, e.g. avidin, specific antibodies, etc., where the binding partner is conjugated to a detectable label. In the case of amplification the label may be conjugated to one or both of the primers.

Primers or oligonucleotides may also be used for detection of specifically hybridizing nucleic acid molecules in hybridization reactions. When used as probes of hybridization reactions, the primer is usually an oligonucleotide which may be substantially longer. In this case, the term "primer" may be misleading as probes of hybridization reactions are preferably nucleic acid molecule of about 50 to about 2000 bases in length. When used for hybridization reactions such as southern or northern blot reactions, the probe can be an oligonucleotide or primer which are typically in the range of about 15 to 50 bases in length or can be considerably longer and may range from about 50 bases to about 2000 bases. Preferably, an oligonucleotide used for hybridization or amplification is at least 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or at least 50 bases in length. However, probes of at least 100, 150, 200, 250, 300, 350, 400, 450, 500, 550, 600, 650, 700, 750, 800, 850, 900, 950 or at least 1000 bases are preferred according to the present invention. Moreover, according to the particular conditions chosen for hybridization, the oligonucleotide probe may even be several hundred or thousand bases longer. Said oligonucleotide may be composed of DNA or RNA. When used as a hybridization probe, it may be, e.g., desirable to use nucleic acid analogs, in order to improve the stability and binding affinity. The term "nucleic acid" shall be understood to encompass such analogs. A number of modifications have been described that alter the chemistry of the phosphodiester backbone, sugars or heterocyclic bases. Among useful changes in the backbone chemistry are phosphorothioates; phosphorodithioates, where both of the non-bridging oxygens are substituted with sulfur; phosphoroamidites; alkyl phosphotriesters and boranophosphates. Achiral phosphate derivatives include, but are not limited to, 3'-O'-5'-S-phosphorothioate, 3'-S-5'-O-phosphorothioate, 3'-CH2-5'-O-phosphonate and 3'-NH-5'-O-phosphoroamidate. Peptide nucleic acids replace the entire phosphodiester backbone with a peptide linkage. Sugar modifications are also used to enhance stability and affinity. The a-anomer of deoxyribose may be used, where the base is inverted with respect to the natural b-anomer. The 2'-OH of the ribose sugar may be altered to form 2'-O-methyl or 2'-O-allyl sugars, which provides resistance to degradation without comprising affinity. Modification of the heterocyclic bases must maintain proper base pairing. Some useful substitutions include deoxyuridine for deoxythymidine; 5-methyl-2'-deoxycytidine and 5-bromo-2'-deoxycytidine for deoxycytidine. 5-propynyl-2'-deoxyuridine and 5-propynyl-2'-deoxycytidine have been shown to increase affinity and biological activity when substituted for deoxythymidine and deoxycytidine, respectively.

The choice and design of the probe particularly depends on the base composition of the target nucleic acid molecule to which the probe hybridizes. Generally, a probe is designed so that the presence of as few as one mismatch in the base pairing of the probe with the target nucleic acid molecule is detectable. Typically, the nucleotide sequence of such a probe corresponds to a nucleotide sequence in an intron or exon of the FM03 gene, in a region suspected of containing a polymorphism. The term "FM03 gene" refers to the genomic DNA sequence from which wild-type or variant FM03 (poly)peptide is expressed. Preferably, the probe comprises a sequence of wild-type FM03. The person skilled in the art knows various techniques for detecting mismatches in the base pairing of nucleic acid molecules. Preferably, these techniques include, but are not limited to, electrophoretic mobility shift assays, thermal mobility shift assays, [please indicate which other techniques could be used].

The term "detecting" relates to nucleic acid based detection or analysis techniques allowing to determine whether or not the nucleotide sequence is a wild-type or variant nucleotide sequence. Preferably, said detecting is performed by PCR, Southern and Northern Blotting (Sambrook et al., "Molecular Cloning, A Laboratory Manual"; ISBN: 0879695765, CSH Press, Cold Spring Harbor, 2001) but also includes "ligase chain reaction"(LCR, EPA320308), "cyclic probe reaction" (CPR), "strand displacement amplification" (SDA, Walker et al. 1992, Nucleic Acid Res. (7): 1691-1696), "transcription based amplification systems" (TAS, Kwoh et al. 1989, Proc. Nat. Acad. Sci. USA 86: 1173, Gingeras et al., PCT Application WO 88/10315), allele-specific hybridization, allele-specific primer extension, allele-specific oligonucleotide ligation, allele-specific cleavage of a flap probe, Taqman PCR, Pyrosequencing, Snapshot, Restriction Fragment length Polymorphism Analysis (RFLP) or Maldi-Tof.

The specification of the present invention also refers to a method of testing an avian for its predisposition for fishy taint in eggs comprising testing a sample for the presence or absence of a threonine in position 329 of FMO3, wherein an absence of a threonine in said position is indicative for a predisposition to produce fishy tainted eggs or to pass on to the offspring a gene responsible for developing such a phenotype.

The method of testing for an avian's predisposition for fishy taint in eggs may comprise the steps of: (a) contacting the sample with an allele-specific antibody or an allele-specific aptamer; and (b) detecting whether a specific binding of said antibody or aptamer to its antigen or binding partner has occurred.

Also described herein is the use of antibodies specifically binding to FM03 (poly)peptides. Said antibodies may be directed to an epitope of an FM03 (poly)protein comprising a serine but not a threonine at position 329 and which fails to bind to an FM03 (poly)peptide which contains a threonine in said position. This antibody is characterized in not recognizing an FM03 (poly)peptide which contains a threonine in position 329 of the (poly)peptide chain. The term "in position 329" relates to position 329 of SEQ ID NO:25. Also comprised are shorter or longer variants of FM03, including variants with additions, duplications and deletions. In order to determine which amino acid residue of the variant FM03 corresponds to position 329 of SEQ NO:25, the skilled person may perform a sequence alignment with programs such as Bestfit^{®} under the conditions outlined above.

Said antibody may be polyclonal, monoclonal, chimeric, single chain, single chain Fv, human antibody, humanized antibody, or Fab fragment. The antibody may be labeled. Preferred labels for the nucleic acid molecules and proteins mentioned in the present invention's specification are fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the antibody is conjugated to biotin, haptens, etc. having a high affinity binding partner, e.g. avidin, specific antibodies, etc., where the binding partner is conjugated to a detectable label. Alternatively, the label may be a toxin, radioisotope, or fluorescent label. Said antibody may be immobilized to a solid support. Preferably, the solid support may be the surface of a cell, a microtiter plate, beads or the surface of a sensor capable of detecting binding of the antibody or to the antibody.

The term "allele-specific antibody" relates to an antibody capable of selectively binding to a variant of FM03 and of discriminating between wild-type FM03 carrying a threonine in position 329 and variants with other amino acid residues in this position. Preferably, said variant specifically bound by said antibody is an FMO3 variant carrying an amino acid other than a threonine in position 329 of the amino acid sequence. More preferred it is an antibody capable of specifically binding to FM03 with a serine in position 329 of the amino acid sequence, wherein said antibody fails to bind to any other known FMO3 (poly)peptides.

In the method of testing an avian for its predisposition for fishy taint in eggs, the allele-specific antibody is specific to an epitope of FM03 comprising a serine but not a threonine at position 329 and which fails to bind to an FM03 (poly)peptide which contains a threonine in said position and wherein binding of the antibody is indicative of a predisposition for fishy taint in eggs.

Also described herein is a method of testing an avian for its predisposition for fishy taint in eggs, comprising the steps of: (a) preparation of a tissue sample from the subject; (b) determining whether the FM03 gene contains at least one polymorphism selected from the polymorphisms shown it table 2; or (c) determining whether the FM03 protein contains a threonine in position 329 of chicken FM03 or in a homologous position of other avian FM03 (poly)peptides, wherein the absence of a threonine is indicative of a predisposition for fishy taint in eggs. However, the present invention's specification also refers to methods of testing which are based on antibodies specific for epitopes comprising a threonine in position 329 of FM03 wherein said antibodies fail to bind to FM03 variants with other amino acid residues in this position. It goes without saying that the use of such an antibody in a testing method would result in a signal only in the case of wild-type FM03 having a threonine in position 329 which would be indicative of "no fishy taint". In contrast, when using such an antibody the absence of a signal will indicate a predisposition for fishy taint.

In a preferred embodiment of the present invention, the sample is isolated from egg, semen, blood, feather, skin or sputum of an avian. However, according to the present invention any sample may be used for testing an avian's predisposition for fishy taint in eggs.

The present invention also relates to a method for selecting an avian or part of an avian, including their semen or eggs, comprising the steps of: (a) testing for a predisposition for fishy taint in eggs comprising the steps of the method of the present invention; (b) selecting the avian based on the information derived from said testing; and (c) optionally, using said information for further breeding considerations. Said breeding considerations may be breeding management considerations such as the propagation, culling or feeding of animals.

In another preferred embodiment of the present invention the avian is selected from the group consisting of chicken, turkey, duck, goose, quail, pheasants, parrots, finches, hawks, crows and ratites including ostrich, emu and cassowary.

The present invention also relates to the use of a nucleic acid molecule of the FM03 gene for testing an avian for its predisposition for fishy taint in eggs characterized by the presence of a serine codon at position 329 of chicken FMO3 as shown in SEQ ID NO:25 or in a homologous position of other avian (poly)peptides wherein said nucleic acid of the FM03 gene is a probe or primer comprising at least 12 nucleotides capable of hybridizing under stringent conditions to the nucleic acid molecule of any one of SEQ ID NOs: 1 to 5 or a fragment or variant thereof, wherein the variant contains at least the polymorphism in position 694 of SEQ ID NO: 3 as shown in table 2.

As used herein, the term "a nucleic acid molecule of the FM03 gene" relates to the genomic DNA of FM03 including introns, exons and regulatory sequences. Preferably, said term also comprises sequences located up to 2000bp upstream or downstream the gene encoding FM03. Also comprised are transcribed and spliced mRNA or reverse-transcribed cDNA. For the purpose of this specification, any of these nucleic acid molecules are to be regarded as nucleic acid molecules of the FMO3 gene. Said nucleic acid molecule may be of wild-type or may contain one or more mutations. They may comprise SEQ ID NOs: 1 to 5 or a fragment thereof; or (b) the nucleic acid molecule of SEQ ID NOs: 1 to 5 having at least one polymorphism selected from the polymorphisms as shown in table 2.

In a preferred embodiment of the present invention's use, the probe is the primer of the present invention.

Finally, the present invention relates to a kit for the detection of animals having a predisposition for fishy taint in eggs, comprising the primer of the present invention in one or more container. In addition, the kit may contain instructions for use.

The figures show:

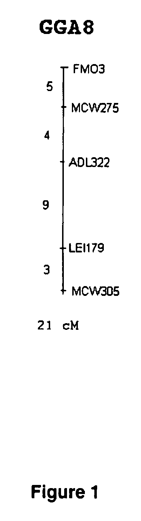

**Figure 1: Chicken chromosome 8 linkage map**. The distal end of GGA8 is shown with the distances between used markers in Kosambi cM (CRIMAP, sex-averaged).
**Figure 2: A schematic representation of the chicken FM03 gene.** The exons are shown as open boxes and labelled by exon number following the numbering of human FM03 exons (non-sequenced untranslated regions are shown in grey). Lines between the boxes indicate intron sequences: the line is interrupted when all of the intron was not sequenced and the length of the intron is undetermined. The locations of primers used for amplification and sequencing (SEQ ID Nos: 6 to 21) are indicated by numbered arrows. Refer to SEQ ID Nos 1 to 5 for detected polymorphisms (listed variations) in the sequence. SEQ ID NOs 22 to 24 are primers used for minisequencing/primer extension assays.

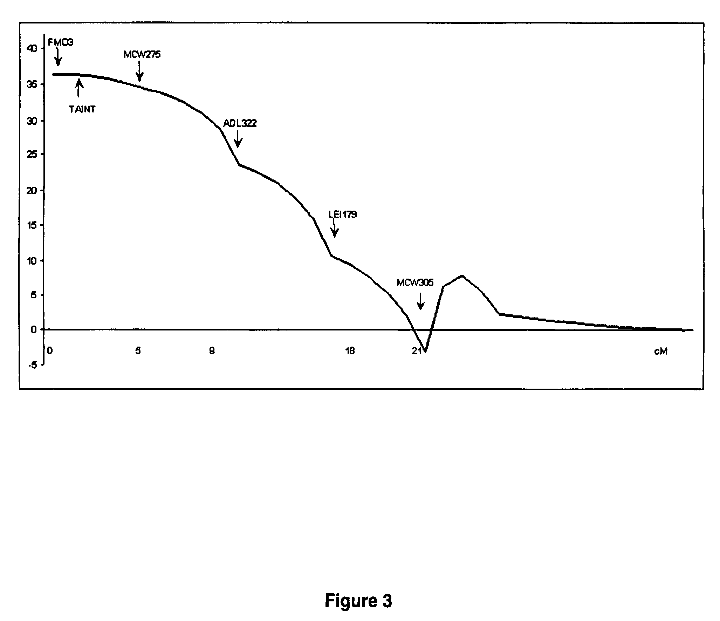

**Figure 3**: Lod score profile obtained for tainting eggs (organoleptic scoring) after rapeseed feeding challenge along the chromosome 8 linkage map using FASTLINK (Cottingham et al. 1993, Schäffer et al. 1994) under the assumption of the trait being autosomal recessive.

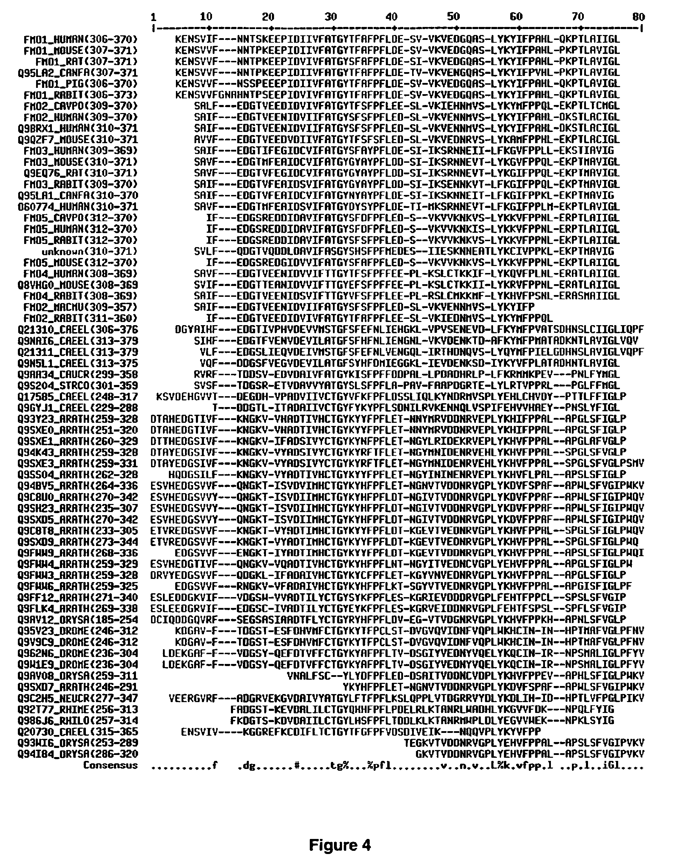

**Figure 4:** Multiple alignment of GenBank protein sequences with >90% similarity to the chicken *FMO3*-gene sequence in the region of 310 - 371 aa. The alignment shows the evolutionary conservation of the -FATGY- motif in all mammalian FMO isoforms, and the -TG- motif in all sequences. The chicken sequence (unknown 310-371) containing the mutation T329S is marked "unknown (310-371)".

The examples illustrate the invention:

### Examples 1: Particular methods and material used in the Examples

**Mapping populations** *Half-sib design at MTT, Jokioinen.* A mapping population was created by an optimised mating plan. Lohmann White Leghorn line 55 and Brown line 88 were selected as parental lines and crossed reciprocally. In the F₁-generation, line 58 consisted of mating of line 5 males to line 8 females. The reciprocal line 85 was composed of line 5 females paired with line 8 females. Line 58 included 122 males and 590 females, and the reciprocal line 85 included 32 males and 163 females. From F₁, all line 85 males and 33 random selected males from line 58 were genotyped with 11 microsatellite markers in order to identify informative matings. Since genotyping results were only available for male individuals, genotype probabilities were derived for the F₁-females. The genotype probabilities of the female offspring were calculated according of known paternal genotype and the maternal genotype probabilities. Thus genotype probabilities for each full sister groups were identical. Fully informative matings were gained when the crossed male and female did not share any alleles. The examination of fully informative matings was done over all studied 11 loci and summed up of all genotypes per males compared with a group of full sister females. That sum was used as an F₁-male informativeness ranking parameter. The F₂-generation was generated from the most informative matings of F₁ individuals in order to maximize the power for linkage analysis. Twenty best males were selected with three most informative mating partners. Each male was mated to three different full sister pairs, altogether 6 females.

Four hatches consisted of 15-20 offspring from 3 full sister pairs, i.e. 90-120 offspring in each half sib family. Availability of single performance testing cages limited flock size from 4463 to 2009 F₂ hens, which were chosen for the mapping population. Ten half sister families from each reciprocal cross were chosen.

**Line cross design at Cuxhaven (confirmation study):** To identify genetic polymorphisms linked to egg taint, a second F₂-cross was set up. In the F₀-generation twenty-one Rhode Island Red hens, which were specifically selected for laying "smelling" eggs were crossed with five White Leghorn males assumed to be "Non-Tainters".

Fifty-four animals formed the F₁-generation. 9 F₁ males were crossed to 45 females to generate the F₂-offspring.

The F₂-generation resulted in forty-three full-sib families including 457 hens. The number of female offspring was about ten in each family.

Treatment of the animals: To make sure that enough precursors of TMA are available a choline loaded feed mixture was fed (5130g choline/kg feed) to the F₂-generation. Choline is a precursor of TMA. Hens which are not able to deal with high amounts of TMA will lay tainted eggs.

A certain time table of feeding choline was set up as it is shown below.

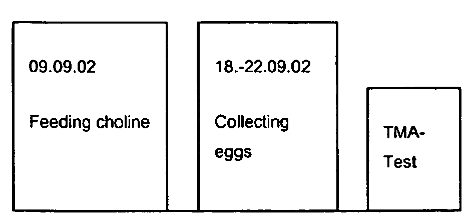

Four organoleptical examinations were done including all F2-female offspring to identify "tainting" hens. Out of the 457 hens 85 tainters were organoleptically identified. For comparison, 85 "Non-Tainters" were selected.

The TMA-N content in the egg yolk of these hens was measured using the TMA-Test as described.

**Determining phenotypes**: Organoleptic observation (Jokioinen mapping population) The F₂ -hens were fed with a feed containing 10% rapeseed meal for two weeks at the age of 50 - 54 weeks. The scoring of the taint was done subsequently by subjective olfactory testing for three consecutive days. Each egg was scored on the scale 1-5, according to the fishy odour, 1 being odorless and 2-5 expressing fishy odour with increasing intensity. For each individual hen, the scores of its eggs during the testing period were amalgamated to an average score.

**Chemical determination of Trimethylamine in egg yolks:** For the conformation study (second crosl-line experiment) a method to quantify the TMA-N content in egg yolks was developed according to Dyer *et al.* (1945) (Brettschneider, J.; not published).

Sample preparation for the photometer measurement involved splitting of the egg into two parts: the egg yolk and the egg white. TMA was extracted from the egg yolk with a 10 % trichloracetic-acid: 15g of egg yolk was mixed with trichloracetic-acid to an end volume of 30ml. After 10 hours the mixture was filtered.

To 2ml of the filtrate, 1,5ml of a 50% potassium hydroxide solution and 0,5ml of a 10% formaldehyde solution was added. Both are important for the stability of the method. To extract TMA out of the reaction mixture 5ml Toluol were pipetted as an cleaning solvent.

After two hours of shaking, 2,5ml of the toluol phase was taken and the same amount of picric-acid was added. After adding the picric acid the yellow colour of trimethylamine picrate is measurable with the photometer.

*Calibration curve:* An analysis of a standard-solution with 0,1mg TMA/ml was used to get series of calibration curves in the range from 0 to 24 µg TMA-N/ml. The curve developed was quite linear to the measured extinction values.

**Preparation of DNA samples:** Each pool contained 20 individuals from the phenotypic extremes for each trait within each half-sib family. From each hen to be included in the pools 20 µl of whole blood was taken in order to prepare a pooled blood sample. DNA was extracted from the pool with the same procedure as from individual samples (Tuiskula-Haavisto et al. 2002). Decision on which individuals were included in each pool was made according to their corrected phenotypic values (average score per individual hen).

**Genotyping and map construction:** Microsatellite markers MCW275, ADL322, LEI179, MCW305 and the SNP markers SNP13 and SNP7 were genotyped for all individuals in the five half-sib families segregating for taint (F1 sires 23163, 23169, 23320, 56323 and 56826). PCR conditions for the microsatellite markers were as published (http://www.zod.wau.nl/vf/ and http://www.ri.bbsrc.ac.uk/cgi-bin/mapviewer?species=chicken). The SNP13 (G to C change within the intron between exons 5 and 6) and SNP7 (A to T change in exon 7) were detected using minisequencing (SnuPe Genotyping, Amersham Biosciences). An amplified PCR product of the region (SNP13: with primers SEQ ID Nos 12 and 13;or SNP7: with primers SEQ ID NOs 16 and 17) was used as a template in a thermally cycled minisequencing reaction with dye-labelled terminators and sequence specific primer ending at the polymorphic base (SNP13: 5'- TGA GGC AGG GTT GAG CCA CC-SEQ ID NO: 23; or SNP7: 5'-GGA AAG GAG TGA GAG TAA CCA G - SEQ ID NO: 24). Excess of deoxynucleotides and primers were removed with specific enzymatic treatment of the reaction product before minisequencing (ExoSap-IT^{™} Amersham Biosciences). Unincorporated dye-labelled terminators were subsequently removed by filtration (AutoSeq^{™}96 plates, Amersham Biosciences). Reaction products were detected on the capillary DNA electrophoresis instrument (MegaBACE, Amersham Biosciences). Analysis was based on allele signal intensity and relative allele mobility with MegaBACE SNP Profiler, version 1.0 (Molecular Dynamics 2001). The linkage map for the markers was constructed using the program package CRIMAP (Green et al. 1990), options BUILD and FLIPS.

**Sequencing the coding portion of the *flavin containing monooxygenase 3 gene (FMO3)* from chicken genomic DNA:**To develop primers that would allow to amplify and sequence the entire FM03 coding sequence from chicken genomic DNA, information on the chicken cDNA and human genomic FM03 was used (Chiu 2002, Dolphin et al. 1997). Exonic primers were designed based on the complete chicken FMO3 cDNA sequence (gi: 18873598, published in February 2002) and the genomic sequence of the human *FMO3* gene (gi: 1209696), either predicted to flank exon-intron boundaries or to amplify exons assuming conservation of exon composition between human and chicken. Based on newly generated intron sequence, new primers were developed to amplify and sequence most of the coding regions of FM03. The amplified fragments were sequenced using the amplification primers as forward and reverse sequencing primers using standard procedures (DYEnamic^{™} ET dyeterminator Kit and MegaBACE, Amersham Biosciences). The primers are given in the sequence listing (SEQ ID NOs 6 - 21).

**Linkage analysis of the disorder**: The linkage analysis of the trait was carried out with FASTLINK (version 4.1 P, Cottingham et al. 1993, Schäffer et al. 1994) using the assumption of the disorder (tainting eggs after feeding challenge) being autosomal recessive. All males and hens not scored for taint (dams) were coded unknown for their affection status. Options used for the analysis were MLINK for two-point analysis and ILINK for multipoint analysis. The most probable location of the disorder locus was determined with LINKMAP where the location scores of the trait were calculated against the fixed map of the other loci in the linkage map (in Figure 1).

### Example 2: Linkage mapping of the FM03 gene in chicken

The location of the FM03 gene in chicken was determined by linkage mapping. Primers were designed to sequence the introns between exon 5 and exon 6 (81 bp) and between exon 6 and exon 7 (110 bp). By sequencing several individuals of the mapping population, six polymorphic sites were identified in that area (SEQ ID NO 3). By using one of these as a marker (SNP13) in linkage analysis, it was possible to show that FM03 in chicken maps to the distal part of chromosome 8 (Figure 1). FM03 maps 5 cM (Kosambi, sex-averaged) distal to the end of the microsatellite linkage map.

### Example 3: Linkage mapping of the trait

Two-point linkage analysis of the disorder (MLINK, under the assumption of the trait being autosomal recessive) against the genotyped markers on distal GGA8 gave the highest probability (LOD score 37.62) of linkage to the marker FMO (SNP marker SNP13) with the recombination fraction 0.0 (Table 3).

**Table 3. Two-point LOD-scores for linkage between taint and markers on GGA8. The highest LOD score for each pairwise combination (at the most probable recombination fraction θ) is shown in bold.**

| Marker θ | 0.0 | 0.01 | 0.05 | 0.1 | 0.2 | 0.3 | 0.4 |
|---|---|---|---|---|---|---|---|
| FM03 | **37.62** | 37.23 | 34.99 | 31.44 | 23.13 | 14.01 | 5.00 |
| MCW275 | 5.79 | 5.87 | **5.95** | 5.64 | 4.36 | 2.70 | 1.00 |
| ADL322 | 15.89 | 16.51 | **17.36** | 16.81 | 13.52 | 8.68 | 3.23 |
| LEI179 | 6.73 | 7.30 | 8.48 | **8.67** | 7.16 | 4.50 | 1.59 |
| MCW305 | -1.90 | -0.46 | 3.44 | 6.31 | **7.19** | 5.01 | 1.83 |

The best location score (LINKMAP) against the fixed linkage map of the markers for taint was placed close to the *FMO3* gene (Figure 3).

### Example 4: Sequencing of the chicken FMO3 gene

The sequence of the chicken FM03 gene was determined from genomic DNA of several individuals. The sequence information produced comprises the exon - intron structure of the gene in chicken, the sequence of the entire coding sequence and partial intron sequence (Figure 2).

The entire coding sequence of the chicken *FMO3* gene was sequenced from 2 tainting and 2 non-tainting individuals. Eight polymorphisms in the coding sequence were identified, but only one of them resulted in an amino acid change. It is due to a single base change A to T at position nt 1034 in the cDNA sequence (gi: 18873598), resulting in the codon change ACT to TCT and the corresponding T to S amino acid change at position aa 329 in the published amino acid sequence (GenBank AJ431390.1). The amino acid change resides within an evolutionary conserved region containing the FMO-characteristic pentapeptide motif (-FATGY-) in mammals (Sterh et al. 1998, Lattard et al. 2001) and a dipeptide (-TG-) in all lineages since archael divergence (Figure 4). The function of the motif is still unknown, but it has been speculated that it may be a substrate recognition pocket of hydroxylases in micro-organisms *(Escherichia coli, Pseudomonas aeruginosa)* and in mammalian flavin containing dimethylaniline mono-oxygenases including isozymes 1 to 5 of *FMO* (Sterh et al. 1998, Ambrosi et al. 2000). Both the evolutionary conservation of the amino acid and the postulated function of the motif it resides in strongly suggest that the amino acid change itself may lead to a dysfunction in the chicken FM03 gene, thus leading to accumulation of TMA in the egg yolk in individuals homozygous for the mutation.

### Example 5: Association analysis between the genotypes at FM03 and tainting status

To analyse the association between the genotypes at FMO3 and tainting status after feeding challenge, TMA-phenotypes and the amino acid 329 (marker SNP7) genotypes were scored in an independent F₂-cross (confirmation study). In the population TMA contents of egg yolk were measured biochemichally. The phenotypes scored after feeding challenge were classified to two groups, HIGH and LOW, according to the TMA-content in egg yolk. Hens in the LOW -group had TMA-content < 3.1 µg/g, HIGH -group hens had TMA >10 µg/g. Genotyping the SNP7 from 168 F₂-hens showed that all TT -homozygotes had high TMA-content while heterozygotes AT and homozygotes AA expressed low TMA-content in egg yolk.

**Table 4: Observed genotype frequencies in HIGH and LOW groups:**

| TMA-group | Homozygote AA | Heterozygote AT | Homozygote TT | Σ |
|---|---|---|---|---|
| HIGH | 0 | 0 | 54 | 54 |
| LOW | 42 | 72 | 0 | 114 |

**Table 5: Expected genotype frequencies**

| TMA-group | Homozygote AA | Heterozygote AT | Homozygote TT |
|---|---|---|---|
| HIGH | 13.5 | 23.1 | 17.4 |
| LOW | 28.5 | 48.9 | 36.6 |

In the X² -test the P-value (X² =167.51) is < .0001 (the probability to get the observed genotype frequencies by chance is less than 0.01 %). The result shows that there is a highly significant association between the TMA-content of the egg yolk and the T329S amino acid substitution.

### Example 6: Additional confirmation study

With regular diets free of egg taint causing substances (precursors of TMA) no egg taint or significant amounts of TMA can be analysed. For the identification of tainters special diets have to be formulated including increased amount of choline, betaine, rapeseed (glucosinolates, tannins) or fishmeal of poor quality to increase the risk of off flavour in egg yolk. Identification of affected hens has been done so far by organoleptical testing, which is a very subjective method. In this application an objective method has been set up and used to separate unbiased between tainters and non-tainters. Unbiased and reliable phenotypic judgement is the basic input for the molecular study to identify the causative mutation.

Samples of three available commercial brown egg layers (ISA Brown, Tetra, Lohmann Brown) have been used in a challenge feeding trial to identify the frequency of tainters. Despite the different genetic background of the strains the association could be confirmed. The same A to T mutation was found to be fully associated with elevated TMA levels in egg yolk. No other mutations leading to amino acid changes were observed. An identical haplotype was found for a studied stretch of the gene (ranging from exon5 to exon7) analysed in all tainters regardless of breed, indicating a common origin for the mutation. The observed high number of polymorphisms between the tainting and wild-type chromosomes and the presence of the T329S variant in several genetically divergent breeds suggest an ancient origin for the mutation.

**Table 6 : Frequency of the amino acid variants (T329S) in different chicken populations. ISA Brown, Lohmann Brown and Tetra are commercial brown layer lines and Maran is an exotic chicken breed used for niche production in Europe. Samples of Icelandic landrace (native) and three standardized chicken breeds from Europe (Fayoumi, Greed-legged partridge and Transsylvanian naked neck) represent rare breeds The ancestral Red Jungle Fowl (Gallus gallus gallus) and Fayoumi and Icelandic landrace show no presence of the "taint" mutation.**

| Population | n | Allele frequency | |
|---|---|---|---|
| | | T | S |
| *Gallus gallus gallus* | 10 | 1.00 | 0.00 |
| Fayoumi | 10 | 1.00 | 0.00 |
| Green-legged partridge | 8 | 0.81 | 0.19 |
| Icelandic landrace | 10 | 1.00 | 0.00 |
| ISA Brown | 71 | 0.58 | 0.42 |
| Lohmann Brown | 67 | 0.62 | 0.38 |
| Maran | 111 | 0.96 | 0.04 |
| TETRA | 70 | 0.61 | 0.39 |
| Transsylvanian naked neck | 8 | 0.69 | 0.31 |

## Claims

1. A method of testing an avian for its predisposition for fishy taint in eggs comprising analyzing the nucleic acid of a sample comprising the gene encoding FMO3 or a corresponding mRNA for a nucleotide polymorphism which is linked to said predisposition, wherein a difference from the wild-type FMO3 sequence is indicative of fishy taint, said difference being the presence of a serine codon in a position corresponding to position 329 of chicken FMO3 as shown in SEQ ID NO: 25 or in a homologous position of other avian FMO3 (poly)peptides.

2. The method of claim 1, wherein said wild-type FMO3 sequence is represented in part by any one of SEQ ID NOs: 1-5.

3. The method of claim 1 or 2, wherein said testing comprises
(a) hybridizing a probe comprising the polymorphic position under stringent conditions to the FM03 gene or to a transcription product therefrom, wherein
(i) the probe comprising the polymorphic position has at the polymorphic position the sequence of the variant corresponding to a serine codon at position 329 of SEQ ID NO:25 and
(ii) the conditions of hybridization are such that binding can only occur if the sequence of the probe and the target nucleic acid at the polymorphic position are completely complementary, and
(b) detecting whether hybridization has occurred, wherein hybridization of a probe comprising the polymorphic position is indicative for a predisposition for fishy taint in eggs.

4. The method of claim 3, wherein said probe is detectably labeled.

5. The method of claim 3, wherein said probe is labeled with a reporter dye and a quenching dye.

6. The method of any of claims 1 to 5, wherein said testing comprises determining the nucleic acid sequence of at least a portion of said nucleic acid molecule.

7. The method of claim 6, wherein the determination of the nucleic acid sequence is effected by solid-phase minisequencing.

8. The method of any of claims 1 to 7 further comprising, prior to analyzing the nucleic acid, amplification of at least a portion of said nucleic acid.

9. The method of any one of claims 1 to 8, wherein primers consisting of SEQ ID NOs: 6 to 24 are used for amplification.

10. The method of claim 8 or 9 wherein said amplification is effected by or said amplification is the polymerase chain reaction (PCR).

11. The method of any of claims 1 to 10, wherein the nucleic acid is analyzed by the use of:
(a) a primer extension assay;
(b) a Taqman^{R} PCR;
(c) a differential hybridization assay;
(d) an assay which detects allele-specific enzyme cleavage; and/or
(e) allele specific PCR.

12. The method of claim 1, wherein prior to analysing the nucleic acid, at least a portion of said nucleic acid is amplified by PCR, the method comprising cycles of (a) stringent hybridization with at least two primers sufficiently complementary to bind to the target FMO3 DNA under stringent conditions of hybridization, wherein at least one primer comprises the polymorphic position indicated in claim 1, having in the polymorphic position of the FMO3 gene the sequence of a variant with a serine codon in a position corresponding to position 329 of SEQ ID NO: 25 and not of wild-type FM03; and (b) strand elongation and denaturing, wherein (i) a failure to generate a PCR product is indicative for a predisposition for eggs with no fishy taint and (ii) the generation of a PCR product is indicative for a predisposition for eggs with fishy taint.

13. The method of claim 1, wherein prior to analysing the nucleic acid, at least a portion of said nucleic acid is amplified by PCR, the method comprising cycles of (a) stringent hybridization with at least two primers sufficiently complementary to bind to the target FM03 DNA under stringent conditions of hybridization, wherein at least one primer comprises the polymorphic position indicated in claim 1, but having the sequence of wild-type FM03 in said position; and (b) strand elongation and denaturing, wherein (i) a failure to generate a PCR product is indicative for a predisposition for fishy taint in eggs and (ii) the generation of a PCR product is indicative for a predisposition for eggs with no fishy taint.

14. A method for selecting an avian or part of an avian, including their semen or eggs, comprising the steps of:
(a) testing for a predisposition for fishy taint in eggs comprising the steps of the method of anyone of claims 1 to 13;
(b) selecting the avian based on the information derived from said testing; and
(c) optionally, using said information for further breeding considerations.

15. The method of any of claims 1 to 14, wherein the sample is from egg, semen, blood, feather, nail, skin or sputum of an avian.

16. The method of any one of claims 1 to 15, wherein the avian is selected from the group consisting of chicken, turkey, pheasants, duck, goose, quail, parrots, finches, hawks, crows and ratites including ostrich, emu and cassowary.

17. Use of a nucleic acid molecule of the FMO3 gene for testing an avian for its predisposition for fishy taint in eggs **characterized by** the presence of a serine codon at position 329 of chicken FMO3 as schown in SEQ ID NO:25 or in a homologous position of other avian FMO3 (poly) peptides, wherein said nucleic acid molecule of the FM03 gene is a probe or primer comprising at least 12 contiguous nucleotides capable of hybridizing under stringent conditions to the nucleic acid molecule of SEQ ID NOs 1 to 5 or a fragment or variant thereof, wherein the variant contains at least the polymorphism in position 694 of SEQ ID NO: 3 as shown in table 2.

18. A primer suitable for use in detecting the polymorphism at nucleotide position 694 of SEQ ID NO:3, said primer consisting of a nucleotide sequence of about at least 8 to about 30 nucleotides capable of hybridizing under stringent conditions to the nucleotide sequence of SEQ ID NO: 3 and said primer comprising a T at the position corresponding to position 694 of SEQ ID NO:3.

19. A kit for the detection of animals having a predisposition for fishy taint in eggs, comprising the primer of claim 18 in one or more container.

## Patentansprüche

1. Verfahren zum Testen eines Vogels auf seine Prädisposition für Fischgeruch in Eiern, umfassend das Analysieren der Nukleinsäure einer Probe, welche das FM03 kodierende Gen oder eine entsprechende mRNA enthält, auf einen Nukleotidpolymorphismus, der mit der Prädisposition gekoppelt vorkommt, wobei ein Unterschied zur Wildtyp FM03-Sequenz auf Fischgeruch hinweist und wobei der Unterschied das Vorhandensein eines Serinkodons in einer Position ist, die der Position 329 des Hühner FM03 entspricht, wie in SEQ ID NO: 25 gezeigt, oder in einer homologen Position eines FM03 (Poly)peptides von anderen Vögeln.

2. Verfahren nach Anspruch 1, wobei die Wildtyp FMO3-Sequenz zum Teil durch eine der SEQ ID NOs: 1 bis 5 dargestellt ist.

3. Verfahren nach Anspruch 1 oder 2, wobei das Testen beinhaltet
(a) Hybridisieren einer Sonde, welche die polymorphe Position enthält, unter stringenten Bedingungen an das FM03-Gen oder an eines seiner Transkriptionsprodukte, wobei
(i) die Sonde, die die polymorphe Position enthält, an der polymorphen Position die Sequenz der Variante hat, die einem Serinkodon an Position 329 der SEQ ID NO: 25 entspricht und
(ii) die Hybridisationsbedingungen so sind, dass eine Hybridisierung nur dann erfolgt, wenn die Sequenz der Sonde und der Zielnukleinsäure an der polymorphen Position vollständig komplementär sind, und
(b) Nachweis ob eine Hybridisierung stattgefunden hat, wobei eine Hybridisierung einer Sonde, die die polymorphe Position enthält, auf eine Prädisposition für Fischgeruch in Eiern hinweist.

4. Verfahren nach Anspruch 3, wobei die Sonde nachweisbar markiert ist.

5. Verfahren nach Anspruch 3, wobei die Sonde mit einem Reporterfarbstoff und einem Quenching-Farbstoff markiert ist.

6. Verfahren nach einem der Ansprüche 1 bis 5, wobei das Testen das Bestimmen der Nukleinsäuresequenz von wenigstens einem Teil des Nukleinsäuremoleküls umfasst.

7. Verfahren nach Anspruch 6, wobei das Bestimmen der Nukleinsäuresequenz durch Festphasen-Minisequenzierung bewirkt wird.

8. Verfahren nach einem der Ansprüche 1 bis 7, des weiteren umfassend das Amplifizieren mindestens eines Teiles der Nukleinsäure vor der Analyse der Nukleinsäure.

9. Verfahren nach einem der Ansprüche 1 bis 8, wobei Primer zur Amplifikation verwendet werden, die aus den SEQ ID NOs: 6 bis 24 bestehen.

10. Verfahren nach Anspruch 8 oder 9, wobei die Amplifikation durch die Polymerasekettenreaktion (PCR) erfolgt, oder wobei die Amplifikation die Polymerasekettenreaktion (PCR) ist.

11. Verfahren nach einem der Ansprüche 1 bis 10, wobei die Nukleinsäure unter Verwendung von:
(a) einem Primerverlängerungsassay;
(b) einer Taqman^{R} PCR;
(c) einem differentiellen Hybridisierungsassay;
(d) einem Test, welcher Allel-spezifische Enzymspaltung nachweist; und/oder
(e) Allel-spezifischer PCR;
analysiert wird.

12. Verfahren nach Anspruch 1, wobei vor der Analyse der Nukleinsäure mindestens ein Teil der Nukleinsäure mittels PCR amplifiziert wird, wobei das Verfahren Zyklen umfasst von (a) stringenter Hybridisierung mit mindestens zwei Primern, die ausreichend komplementär sind, um die Ziel-FM03 DNA unter stringenten Hybridisierungsbedinungen zu binden, wobei wenigstens ein Primer die wie in Anspruch 1 dargestellte polymorphe Position umfasst, die in der polymorphen Position des FM03-Gens die Sequenz einer Variante hat, mit einem Serinkodon in einer Position, die der Position 329 der SEQ ID NO: 25 entspricht, und die nicht die Sequenz des Wildtyp FM03 hat; und (b) Strangverlängerung und -denaturierung, wobei (i) das Ausbleiben des Erhalts eines PCR Produktes auf eine Prädisposition für Eier ohne Fischgeruch hinweist und (ii) der Erhalt eines PCR Produktes auf eine Prädisposition für Eier mit Fischgeruch hinweist.

13. Verfahren nach Anspruch 1, wobei vor der Analyse der Nukleinsäure mindestens ein Teil der Nukleinsäure mittels PCR amplifiziert wird, wobei das Verfahren Zyklen umfasst von (a) stringenter Hybridisierung mit mindestens zwei Primern, die ausreichend komplementär sind, um die Ziel-FM03 DNA unter stringenten Hybridisierungsbedingungen zu binden, wobei mindestens ein Primer die in Anspruch 1 dargestellte polymorphe Position umfasst, wobei jedoch diese Position die Sequenz des Wildtyp FM03 hat; und (b) Strangverlängerung und -denaturierung, wobei (i) das Ausbleiben des Erhalts eines PCR Produktes auf eine Prädisposition für Fischgeruch in Eiern hinweist und (ii) der Erhalt eines PCR Produktes auf eine Prädisposition für Eier ohne Fischgeruch hinweist.

14. Verfahren zur Selektion eines Vogels oder von Teilen eines Vogels, einschließlich dessen Samen oder Eier, umfassend die Schritte:
(a) Testen auf eine Prädisposition für Fischgeruch in Eiern, umfassend die Schritte des Verfahrens nach einem der Ansprüche 1 bis 13;
(b) Selektieren des Vogels basierend auf den aus diesem Test abgeleiteten Informationen; und
(c) gegebenenfalls Verwendung dieser Informationen für weitere Züchtungsentscheidungen.

15. Verfahren nach einem der Ansprüche 1 bis 14, wobei die Probe vom Ei, Samen, Blut, Feder, Nagel, Haut oder Auswurf eines Vogels stammt.

16. Verfahren nach einem der Ansprüche 1 bis 15, wobei der Vogel ausgewählt ist aus der Gruppe bestehend aus Hühnern, Truthähnen, Fasanen, Enten, Gänsen, Wachteln, Papageien, Finken, Falken/Habichten, Krähen und Laufvögeln inklusive Strauß, Emu und Kasuar.

17. Verwendung eines Nukleinsäuremoleküls des FM03-Gens zum Testen eines Vogels auf seine Prädisposition für Fischgeruch in Eiern, **dadurch gekennzeichnet, dass** ein Serinkodon an der Position 329 des Hühner FM03 vorkommt, wie in SEQ ID NO: 25 gezeigt, oder in einer homologen Position eines FM03 (Poly)peptides eines anderen Vogels, wobei das Nukleinsäuremolekül des FM03-Gens eine Sonde oder ein Primer ist, umfassend mindestens 12 zusammenhängende Nukleotide, welche/r in der Lage ist unter stringenten Bedingungen an das Nukleinsäuremolekül der SEQ ID NO: 1 bis 5 zu hybridisieren, oder an ein Fragment oder eine Variante davon, wobei die Variante mindestens den Polymorphismus in Position 694 der SEQ ID NO: 3 enthält, wie in Tabelle 2 gezeigt.

18. Primer, der für die Verwendung zum Nachweis des Polymorphismus an der Nukleotidposition 694 der SEQ ID NO: 3 geeignet ist, wobei der Primer aus einer Nukleotidsequenz von etwa mindestens 8 bis etwa 30 Nukleotiden besteht, die unter stringenten Bedingungen an die Nukleotidsequenz der SEQ ID NO: 3 hybridisieren kann und wobei der Primer ein T an der Position enthält, die der Position 694 der SEQ ID NO: 3 entspricht.

19. Kit zur Ermittlung von Tieren, welche eine Prädisposition für Fischgeruch in Eiern haben, umfassend die Primer nach Anspruch 18 in einem oder mehreren Behältern.

## Revendications

1. Méthode pour évaluer un aviaire pour sa prédisposition à une mauvaise odeur dans les oeufs, comprenant l'analyse de l'acide nucléique d'un échantillon comprenant le gène codant FM03 ou un ARNm correspondant pour un polymorphisme nucléotidique qui est lié à ladite prédisposition, dans lequel une différence par rapport à la séquence FMO3 de,type sauvage est indicative d'une mauvaise odeur, ladite différence correspondant à la présence d'un codon sérine à une position correspondant à la position 329 de FMO3 de poulet tel que représenté dans SEQ ID NO: 25 ou dans une position homologue d'autres (poly)peptides FM03 aviaires.

2. Méthode selon la revendication 1, dans laquelle ladite séquence FM03 de type sauvage est représentée en partie par l'une quelconque de SEQ ID NO : 1 à 5.

3. Méthode selon la revendication 1 ou 2, dans laquelle ladite évaluation comprend :
(a) l'hybridation d'une sonde comprenant la position polymorphique dans des conditions stringentes au gène FM03 ou à un produit de transcription issu de celui-ci, où
(i) la sonde comprenant la position polymorphique a, au niveau de la position polymorphique, la séquence du variant correspondant à un codon sérine au niveau de la position 329 de SEQ ID NO : 25, et
(ii) les conditions d'hybridation sont telles qu'une liaison ne peut avoir lieu que si la séquence de la sonde et l'acide nucléique cible au niveau de la position polymorphique sont complètement complémentaires, et
(b) la détection de l'occurrence d'une hybridation, une hybridation d'une sonde comprenant la position polymorphique étant indicative d'une prédisposition pour une mauvaise odeur dans les oeufs.

4. Méthode selon la revendication 3, dans laquelle la sonde est marquée de manière détectable.

5. Méthode selon la revendication 3, dans laquelle ladite sonde est marquée avec un colorant rapporteur et un colorant d'extinction.

6. Méthode selon l'une quelconque des revendications 1 à 5, dans laquelle ladite évaluation comprend la détermination de la séquence d'acide nucléique d'au moins une partie de ladite molécule d'acide nucléique.

7. Méthode selon la revendication 6, dans laquelle la détermination de la séquence d'acide nucléique est réalisée par miniséquençage en phase solide.

8. Méthode selon l'une quelconque des revendications 1 à 7, comprenant en outre, avant analyse de l'acide nucléique, une amplification d'au moins une partie dudit acide nucléique.

9. Méthode selon l'une quelconque des revendications 1 à 8, dans laquelle des amorces constituées de SEQ ID NO : 6 à 24 sont utilisées pour une amplification.

10. Méthode selon la revendication 8 ou 9, dans laquelle ladite amplification est réalisée par, ou ladite amplification est, la réaction d'amplification en chaîne par polymérase (PCR).

11. Méthode selon l'une quelconque des revendications 1 à 10, dans laquelle l'acide nucléique est analysé par l'utilisation de :
(a) une analyse par extension d'amorce ;
(b) une PCR Taqman^{R}
(c) une analyse par hybridation différentielle ;
(d) une analyse qui détecte un clivage enzymatique spécifique d'un allèle ; et/ou
(e) une PCR spécifique d'un allèle.

12. Méthode selon la revendication 1, dans laquelle, avant analyse de l'acide nucléique, au moins une partie dudit acide nucléique est amplifiée par PCR, la méthode comprenant des cycles de (a) hybridation stringente avec au moins deux amorces suffisamment complémentaires pour se lier à l'ADN FM03 cible dans des conditions stringentes d'hybridation, dans laquelle au moins une amorce comprend la position polymorphique indiquée dans la revendication 1, ayant au niveau de la position polymorphique du gène FM03 la séquence d'un variant avec un codon sérine à une position correspondant à la position 329 de SEQ ID NO : 25 et non un FM03 de type sauvage ; et (b) une élongation et dénaturation de brin, dans laquelle (i) l'impossibilité de générer un produit de PCR indique une prédisposition des oeufs à l'absence de mauvaise odeur et (ii) la génération d'un produit de PCR indique une prédisposition des oeufs à une mauvaise odeur.

13. Méthode selon la revendication 1, dans laquelle, avant analyse de l'acide nucléique, au moins une partie dudit acide nucléique est amplifiée par PCR, la méthode comprenant des cycles de (a) hybridation stringente avec au moins deux amorces suffisamment complémentaires pour se lier à l'ADN FM03 cible dans des conditions stringentes d'hybridation, dans laquelle au moins une amorce comprend la position polymorphique indiquée dans la revendication 1, mais ayant la séquence de FM03 de type sauvage au niveau de ladite position ; et (b) une élongation et dénaturation de brin, dans laquelle (i) l'impossibilité de générer un produit de PCR indique une prédisposition à une mauvaise odeur dans les oeufs et (ii) la génération d'un produit de PCR indique une prédisposition des oeufs à l'absence de mauvaise odeur.

14. Méthode pour sélectionner un aviaire ou une partie d'un aviaire, y compris leurs semences ou oeufs, comprenant les étapes de :
(a) évaluation d'une prédisposition à une mauvaise odeur dans des oeufs, comprenant les étapes de la méthode selon l'une quelconque des revendications 1 à 13 ;
(b) la sélection de l'aviaire sur la base de l'information dérivée de ladite évaluation ; et
(c) éventuellement, l'utilisation de ladite information pour des considérations de reproduction supplémentaires.

15. Méthode selon l'une quelconque des revendications 1 à 14, dans laquelle l'échantillon provient d'un oeuf, semence, sang, plume, ongle, peau ou crachat d'un aviaire.

16. Méthode selon l'une quelconque des revendications 1 à 15, dans laquelle l'aviaire est choisi dans le groupe constitué de poulet, dinde, faisans, canard, oie, caille, perroquets, fringillidés, aigle, corbeau et ratites incluant l'autruche, émeu et casoar.

17. Utilisation d'une molécule d'acide nucléique du gène FM03 pour évaluer un aviaire pour sa prédisposition à une mauvaise odeur dans des oeufs, **caractérisée par** la présence d'un codon sérine au niveau de la position 329 de FM03 de poulet tel que représenté dans SEQ ID NO : 25 ou au niveau d'un position homologue d'autres (poly)peptides FM03 aviaires, dans laquelle ladite molécule d'acide nucléique du gène FM03 est une sonde ou amorce comprenant au moins 12 nucléotides contigus capable de s'hybrider dans des conditions stringentes à la molécule d'acide nucléique de SEQ ID NO : 1 à 5 ou un fragment ou variant de celles-ci, le variant contenant au moins le polymorphisme au niveau de la position 694 de SEQ ID NO : 3 tel que représentée dans le tableau 2.

18. Amorce adaptée à une utilisation pour la détection du polymorphisme au niveau de la position nucléotidique 694 de SEQ ID NO : 3, ladite amorce étant constituée d'une séquence nucléotidique d'environ au moins 8 à environ 30 nucléotides capables de s'hybrider dans des conditions stringentes à la séquence nucléotidique de SEQ ID NO : 3 et ladite amorce comprenant une T au niveau de la position correspondant à la position 694 de SEQ ID NO : 3.

19. Trousse pour la détection d'animaux ayant une prédisposition à une mauvaise odeur dans les oeufs, comprenant l'amorce selon la revendication 18 dans un ou plusieurs récipients.


### Sequence listing

<110> LOHMANN TIERZUCHT GmbH
<120> MARKER ASSISTED SELECTION OF CHICKEN AGAINST FISHY TAINT IN FRESH EGGS
<130> G3223 EP
<150> EP 03 02 1556.0
   <151> 2003-09-24
<160> 25
<170>
<210> 1
   <211> 135
   <212> DNA
   <213> Gallus gallus
<220>
   <221> primer_bind
   <222> (1)..(20)
   <223> CF3ex1for primer bind site
<220>
   <221> primer_bind
   <222> (116)..(135)
   <223> CF3ex2rev primer bind site
<220>
   <221> variation
   <222> (90)..(90)
   <223> C-> T variation in exon 1-2
<400> 1
   ATGGTGCGACGCGTGGCTGTGGTGGGCGCGGGCATCAGCGGGTTGGCGGCCACCAAGTGC 60
   TGCCTGGAAGAGGGACTGGAGCCCACCTGCTTTGAGCAGAGCGAGGACATCGGGGGGCTC 120
   TGGCGCTACACGGAA 135
<210> 2
   <211> 714
   <212> DNA
   <213> Gallus gallus
<220>
   <221> primer_bind
   <222> (1)..(20)
   <223> CF3ex3for primer bind site
<220>
   <221> primer_bind
   <222> (626)..(645)
   <223> CF3ex4rev primer bind site
<220>
   <221> primer_bind
   <222> (497)..(511)
   <223> CF3ex4for primer bind site
<220>
   <221> primer_bind
   <222> (695)..(714)
   <223> CF3in4rev primer bind site
<220>
   <221> variation
   <222> (86)..(86)
   <223> C-> T variation in exon 3
<220>
   <221> variation
   <222> (689)..(689)
   <223> G-> A variation in intron 3
<400> 2
   CAGAGGAAGGCAGAGCTAGCATCTACCGCACTGTCTTCACCAATTCCTGCAAGGAGATGA 60
   TGTGCTACCCCGACTTCCCCTTCCCCGATGACTACCCCAACTACATACACAACGCACGGC 120
   TCCATAAGTACATCCGTGACTACGCACAGCATTTTGACCTCCTCCGGCACATCCGCTTCA 180
   AGGTAGGAGGGATGGACTTTGCTGCTGTGTCTCAGAGAAAATCCTGCGGCAGACATCCTG 240
   AGGGAAAGTCTTGTTGGTTTTGTTAAATTGGGTCTCACCAGCTGCTGGTTGGGTAGTGTC 300
   AAATGATCAGTCATCACGGGGAAACCATTGCTTCTGAGGGTGGAAAATCACGTGGAATGG 360
   GAGGACACCAGTGGGTTTTCTCTATCCAAAATAATTGCCAAGTCTGTGACCTTGTCTCTG 420
   GTGGGGAAAAGGACAGAGCCTGCCTGGCTGCAGTGGGGTACCCGGAGCACTTCCCAAACC 480
   TTTATCCCTTTCCTTTAAGACCACAGTCACCAAGATAAGGAAGCGCCCTGACTTCAGTGC 540
   TACGGGGCAGTGGGAGGTGGTTACACGGAGTGACGGGAAGGAAGAGGCAGCGGTTTTTGA 600
   TGCTGTTATGGTTTGCACTGGGCACCATGTCTACCCAAACCTCCCCCTTGCCCACTTCCC 660
   AGGTAAGCTATATGAACGTGATTCAGCCGTCACACAGCCAGGTGTGCACTTTAC 714
<210> 3
   <211> 894
   <212> DNA
   <213> Gallus gallus
<220>
   <221> primer_bind
   <222> (1)..(22)
   <223> CF3ex5for primer bind site
<220>
   <221> primer_bind
   <222> (192)..(211)
   <223> SNP13 reverse primer bind site
<220>
   <221> primer_bind
   <222> (250)..(270)
   <223> CF3ex6rev primer bind site
<220>
   <221> primer_bind
   <222> (250)..(270)
   <223> CF3ex6for primer bind site
<220>
   <221> primer_bind
   <222> (487)..(509)
   <223> SNP14 reverse primer bind site
<220>
   <221> primer_bind
   <222> (616)..(639)
   <223> CF3ex7rev primer bind site
<220>
   <221> primer_bind
   <222> (616)..(637)
   <223> CF3ex7for primer bind site
<220>
   <221> primer_bind
   <222> (875)..(894)
   <223> CF3ex7rev2 primer bind site
<220>
   <221> primer_bind
   <222> (695)..(716)
   <223> SNP7 reverse primer bind site
<220>
   <221> variation
   <222> (98)..(98)
   <223> T -> C variation in exon 5
<220>
   <221> variation
   <222> (101)..(101)
   <223> T-> C variation in exon 5
<220>
   <221> variation
   <222> (184)..(184)
   <223> A-> G variation in intron 4
<220>
   <221> variation
   <222> (189)..(189)
   <223> A-> G variation in intron 4
<220>
   <221> variation
   <222> (191)..(191)
   <223> G-> C variation in intron 4
<220>
   <221> variation
   <222> (249)..(249)
   <223> C->T variation in exon 6
<220>
   <221> variation
   <222> (486)..(486)
   <223> G-> A variation in intron 5
<220>
   <221> variation
   <222> (510)..(510)
   <223> G-> C variation in intron 5
<220>
   <221> variation
   <222> (615)..(615)
   <223> C->T variation in exon 7
<220>
   <221> variation
   <222> (694)..(694)
   <223> A->T variation in exon 7
<400> 3
   GGATAGAGAAGTTTAAAGGTTGCTACTTACACAGCCGGGACTACAAGGAGCCAGAAAAGT 60
   TCAGAGGGAAGAAGGTGCTGGTGGTTGGCTTGGGCAATTCTGGCTGTGACATTGCTGTGG 120
   AGCTCAGCACCGTGGCCTCGCAGGTATGGGGACCTGAACCAGGGCTGGTTGGAGCTGATG 180
   GCCACCACAAGGGTGGCTCAACCCTGCCTCACCATCCCCCCCCAGGTTTACCTGAGCTCC 240
   CGAAGAGGCTCCTGGGTGATGAGCCGTGTCTGGAACTTCGGCTACCCCTGGGACATGCTG 300
   CTCATCACCCGCTTCTGGACGTGGCTGGACAACTTTCTCCCCAAGGCAGTCAGTGACTGG 360
   CTCTATGTCAGGAGCATGAACCAGCAGTACAAACACGAGGATTTTGGCCTAATGCCGGTG 420
   GATGGGTAAGCATGAGGCTATCTGTTCCCAAAGCCAGAAGTGCTGGTCCCAGGCAGGGTG 480
   GTATCGAGCCTGTACAGGGCCAGCAGCAGGAGCCAACTTAGGCTTTTTCCTTGCAGATCA 540
   TCCCGCAGGGAACCGGTGTTGAATGATGACATCCTGAGCCGCATCACCTGTGGTGTGGTG 600
   CTGATAAAGCCAAACGTGAAGGAATTCAGAGAAACATCAGTCTTGTTTCAAGATGGGACT 660
   GTGCAGGACGACCTCGATGCGGTTATCTTTGCCACTGGTTACTCTCACTCCTTTCCTTTC 720
   ATGGAGGATGAATCCATCATCGAAAGCAAGAACAACGAGGCTACCCTCTACAAATGCATT 780
   GTTCCTCCTAAGTTGGAGAAGCCAACCATGGCAGTCATTGGATTGGTCCAGTCCTTTGGA 840
   TCCGCCATCCCGACAGCAGATGTCCAGTGCCGCTGGGCAGTCAAGGTGTTTCAG 894
<210> 4
   <211> 332
   <212> DNA
   <213> Gallus gallus
<220>
   <221> primer_bind
   <222> (1)..(20)
   <223> CF3ex8for primer bind site
<220>
   <221> primer_bind
   <222> (313)..(332)
   <223> CF3ex9rev2 primer bind site
<220>
   <221> variation
   <222> (85)..(85)
   <223> T-> C variation in intron 7
<220>
   <221> variation
   <222> (277)..(277)
   <223> C-> T variation in intron 7
<400> 4
   GGGCTCTGCACTCTCCCCTCAGTCAACGAGATGCTGGAGGACATAGAGGAGAAGAAGAGG    60
   AATAAGATCAGATGGTAGGTGCAGTTCATGGGACGCTGCCCAGGAAGGAGGAGGAAAAAC   120
   CTTGCTGTCTTCTCAGCCTGGGTGGGCTGGATGTGGGCATGGGGAGGGCTGCAGCAGGGG   180
   CAAGCTCCTAGCAAGGAGACCATTCTGAACTGCTCCGTTCATGACCCAGATGAGCAAACC   240
   CAATGGGACCAACACAGGAAGGTCATACCCAGCTTCCTTGGTGTCAGTTCTCAGGTTTCC   300
   ACCCGCCCCAGGGTGGTTGAATTGGGCAAATC                               332
<210> 5
   <211> 335
   <212> DNA
   <213> Gallus gallus
<220>
   <221> primer_bind
   <222> (1)..(20)
   <223> CF3ex9for primer binding site
<220>
   <221> primer_bind
   <222> (316)..(335)
   <223> CF3ex9rev primer bind site
<220>
   <221> variation
   <222> (60)..(60)
   <223> T-> C variation in exon 9
<220>
   <221> variation
   <222> (150)..(150)
   <223> T->C variation in exon 9
<400> 5
   TGGTTCGGGACAAGCAACACGCTGCAGACCGATTATATCACCTACATGAATGAGCTGACT 60
   TCAGCCATCGGTGCGAAGCCCAACGTGCTCAAGCTTCTGCTGACAGACCCACTACTGGCC 120
   CTGAAGGTCTTCTTCGGCCCCTGCACCCCTTACCAGTTCCGCCTGACGGGACCAGGGAAG 180
   TGGAGTGGGGCCAGGAAAGCCATCTTCACCCAGTGGGACCGGACGCTGAAGCCCACGAGG 240
   ACCCGTGAAGCCCCCCCTGCCACCAGTACACTCCCCAGCCTGCTGGTGCTGGGGATGCTC 300
   CTCATCCCCCTCCTCCTCCTCCTCACTGTGCTCTA 335
<210> 6
   <211> 20
   <212> DNA
   <213> Gallus gallus
   <223> CF3ex1for
<400> 6
   ATGGTGCGAC GCGTGGCTGT 20
<210> 7
   <211> 20
   <212> DNA
   <213> Gallus gallus
   <223> CF3ex2rev
<400> 7
   TTCCGTGTAG CGCCAGAGCC 20
<210> 8
   <211> 20
   <212> DNA
   <213> Gallus gallus
   <223> CF3ex3for
<400> 8
   CAGAGGAAGG CAGAGCTAGC 20
<210> 9
   <211> 20
   <212> DNA
   <213> Gallus gallus
   <223> CF3ex4rev
<400> 9
   GGGAGGTTTG GGTAGACATG 20
<210> 10
   <211> 20
   <212> DNA
   <213> Gallus gallus
   <223> CF3ex4for
<400> 10
   CGCTTCAAGA CCACAGTCAC 20
<210> 11
   <211> 20
   <212> DNA
   <213> Gallus gallus
   <223> CF3in4rev
<400> 11
   GTAAAGTGCA CACCTGGCTG 20
<210> 12
   <211> 23
   <212> DNA
   <213> Gallus gallus
   <223> CF3ex5for
<400> 12
   GGGATAGAGA AGTTTAAAGG TTG 23
<210> 13
   <211> 20
   <212> DNA
   <213> Gallus gallus
   <223> CF3ex6rev
<400> 13
   ACACGGCTCA TCACCCAGGA 20
<210> 14
   <211> 20
   <212> DNA
   <213> Gallus gallus
   <223> CF3ex6for
<400> 14
   TCCTGGGTGA TGAGCCGTGT 20
<210> 15
   <211> 23
   <212> DNA
   <213> Gallus gallus
   <223> CF3ex7rev
<400> 15
   GATGTTTCTC TGAATTCCTT CAC 23
<210> 16
   <211> 21
   <212> DNA
   <213> Gallus gallus
   <223> CF3ex7for
<400> 16
   GTGAAGGAAT TCAGAGAAAC A 21
<210> 17
   <211> 19
   <212> DNA
   <213> Gallus gallus
   <223> CF3ex7rev2
<400> 17
   CTGAAACACC TTGACTGCC 19
<210> 18
   <211> 20
   <212> DNA
   <213> Gallus gallus
   <223> CF3ex8for
<400> 18
   GGGCTCTGCA CTCTCCCCTC 20
<210> 19
   <211> 20
   <212> DNA
   <213> Gallus gallus
   <223> CF3ex9rev2
<400> 19
   GATTTGCCCA ATTCAACCAC 20
<210> 20
   <211> 20
   <212> DNA
   <213> Gallus gallus
   <223> CF3ex9for
<400> 20
   TGGTTCGGGA CAAGCAACAC 20
<210> 21
   <211> 20
   <212> DNA
   <213> Gallus gallus
   <223> CF3ex9rev
<400> 21
   TAGAGCACAG TGAGGAGGAG 20
<210> 22
   <211> 20
   <212> DNA
   <213> Gallus gallus
   <223> SNP14 reverse
<400> 22
   CTGCTGGCCC TGTACAGGCT 20
<210> 23
   <211> 20
   <212> DNA
   <213> Gallus gallus
   <223> SNP13 reverse
<400> 23
   TGAGGCAGGG TTGAGCCACC 20
<210> 24
   <211> 22
   <212> DNA
   <213> Gallus gallus
   <223> SNP7 reverse
<400> 24
   GGAAAGGAGT GAGAGTAACC AG 22
<210> 25
   <211> 530
   <212> amino acid
   <213> Gallus gallus
<223> amino acid sequence translation of coding region of chicken *FMO3 gene*
<400> 25